# EUROPEAN PATENT SPECIFICATION

(11) **EP 3 944 838 B1**
(45) Date of publication and mention of the grant of the patent: **04.12.2024**
(21) Application number: 21187456.5
(22) Date of filing: 23.07.2021
(51) Int. Cl.: A61F 2/95, A61F 2/24

(54) **NON-UNIFORM LOADING SYSTEMS AND METHODS FOR IMPLANTABLE MEDICAL DEVICES**
UNGLEICHMÄSSIGE LADESYSTEME UND VERFAHREN FÜR IMPLANTIERBARE MEDIZINISCHE VORRICHTUNGEN
SYSTÈMES ET PROCÉDÉS DE CHARGEMENT NON UNIFORMES POUR DISPOSITIFS MÉDICAUX IMPLANTABLES

(30) Priority: 28.07.2020 US 202063057469 P; 07.07.2021 US 202117369433
(43) Date of publication of application: 02.02.2022
(60) Divisional application: 24209408.4
(73) Proprietor: Medtronic, Inc., Minneapolis, MN 55432 (US)
(72) Inventor: DUNLEA, Jake, Minneapolis (US); LEHMANN, Luke, Minneapolis (US); O'BRIEN, Dermot, Minneapolis (US); MULVIHILL, Bernard Patrick, Minneapolis (US); ANDERSON, Marc A., Minneapolis (US)
(74) Representative: Zimmermann & Partner Patentanwälte mbB

(56) References cited:
- EP-A1- 2 886 082
- WO-A2-2012/116368
- US-A1- 2012 284 986
- US-A1- 2015 081 011
- US-A1- 2019 117 394

## Description

### FIELD

The present technology is generally related to medical devices. And, more particularly, to systems and methods for loading stents, prosthetic heart valves and other implantable medical devices onto delivery systems.

### BACKGROUND

Patients suffering from various medical conditions or diseases may require surgery to install an implantable medical device. For example, valve regurgitation or stenotic calcification of leaflets of a heart valve may be treated with a heart valve replacement procedure. A traditional surgical valve replacement procedure requires a sternotomy and a cardiopulmonary bypass, which creates significant patient trauma and discomfort. Traditional surgical valve procedures may also require extensive recuperation times and may result in life-threatening complications.

One alternative to a traditional surgical valve replacement procedure is delivering implantable medical devices using minimally-invasive techniques. For example, a prosthetic heart valve can be percutaneously and transluminally delivered to an implant location. In such methods, the prosthetic heart valve can be compressed or crimped on a delivery catheter for insertion within a patient's vasculature; advanced to the implant location; and re-expanded to be deployed at the implant location. In this example, a catheter loaded with the prosthetic heart valve in a compressed arrangement can be introduced through an opening in a blood vessel, for example, the femoral artery, aortic artery, or the subclavian artery, and advanced to the heart. At the heart, the prosthetic heart valve can be re-expanded to be deployed at the implant location, *e.g.,* the aortic valve annulus.

In minimally-invasive techniques, it is advantageous to have a small delivery profile for the implantable medical device and delivery system in order to treat a broader range of patient vasculatures. While the profile of the delivery system may be reduced, the given implantable medical device, *e.g.,* a prosthetic heart valve will remain the same size. Accordingly, the reduction in the profile of the delivery system may lead to a higher packing density for the implantable medical device, *i.e.,* the ratio of device volume to available volume. For a prosthetic heart valve, the higher packing density can lead to overlap in the prosthetic heart valve, which is a condition in which portions of the stent or frame of the prosthetic heart valve folds inward in order to fit the reduced space of the delivery system. If this overlap becomes concentrated, the overlap can create elevated crimp strain thereby impacting the structural integrity of the prosthetic heart valve.

US 2019/117394 A1 describes folding patterns and loading funnel for improved transcatheter valve loading forces. US 2012/284986 A1 describes a stent crimping system and method. US 2015/081011 A1 describes systems and methods for loading a valve prosthesis onto a catheter.

### SUMMARY

The techniques of this disclosure generally relate to loading systems for loading an implantable medical device onto a delivery device and converting the implantable medical device from an expanded (uncompressed) arrangement to a compressed (crimped) arrangement.

The first invention relates to a system for transitioning an implantable medical device from an uncompressed arrangement to a compressed arrangement. The system includes an inflow loading assembly configured to compress an inflow portion of the implantable medical device as the implantable medical device is advanced through the inflow loading assembly. The system also includes an outflow loading assembly removably coupled to the inflow loading assembly. The outflow loading assembly is configured to partially compress an outflow portion of the implantable medical device during coupling to the inflow loading assembly. The inflow loading assembly includes one or more biasing features that are configured to asymmetrically compress the inflow portion of the implantable medical device.

In another aspect of the present disclosure, in combination with any of the other aspects herein, the biasing features comprise one or more ridges formed on an interior surface of the inflow loading assembly.

In another aspect of the present disclosure, in combination with any of the other aspects herein, the biasing features comprise one or more bumps formed on an interior surface of the inflow loading assembly.

According to the first invention,
the inflow loading assembly comprises a first portion and a second portion extending therefrom, and wherein at least one of the first portion or the second portion comprises the one or more biasing features.

In another aspect of the present disclosure, in combination with any of the other aspects herein, the first portion has a constant cross-sectional area.

In another aspect of the present disclosure, in combination with any of the other aspects herein, the first portion and a second portion comprise a common continuous interior surface, the common continuous interior surface having a decreasing diameter from an opening of the second portion to a junction of the second portion and the first portion.

In another aspect of the present disclosure, in combination with any of the other aspects herein, the biasing features comprise one or more ridges formed on an interior surface of the inflow loading assembly that extend from an open end of the first portion to an open end of the second portion.

In another aspect of the present disclosure, in combination with any of the other aspects herein, the biasing features comprise one or more bumps formed on an interior surface of the inflow loading assembly that extend from an open end of the first portion to an open end of the second portion.

According to the first invention,
at least one of the first potion or the second portion is formed having a non-circular cross-section.

In another aspect of the present disclosure, in combination with any of the other aspects herein, the system is a loading system for transitioning an implantable medical device, in particular a prosthetic heart valve, from an uncompressed arrangement to a compressed arrangement.

In another aspect, the present disclosure provides a system for percutaneously delivering a prosthetic heart valve, the prosthetic heart valve being radially self-expandable from a compressed arrangement to an uncompressed arrangement. The system includes a delivery having a distal portion and a proximal control handle portion by which the distal portion is effectively controlled. The system also includes a loading system according to the the first invention. The loading system is configured to transition the prosthetic heart valve from the uncompressed arrangement to the compressed arrangement on the distal portion of the delivery device. The loading system includes an inflow loading assembly configured to compress an inflow portion of the prosthetic heart valve as the prosthetic heart valve is advanced through the inflow loading assembly. The inflow loading assembly includes one or more biasing features that are configured to asymmetrically compress the inflow portion of the implantable medical device. The loading system also includes an outflow loading assembly removably coupled to the inflow loading assembly. The outflow loading assembly is configured to partially compress an outflow portion of the prosthetic heart valve during coupling to the inflow loading assembly.

In another aspect of the present disclosure, in combination with any of the other aspects herein, the system is a percutaneous prosthetic heart valve delivery system.

The second invention relates to a crimper for altering an implantable medical device from an uncompressed arrangement to a compressed arrangement. The crimper includes a crimper housing that includes a plurality of crimper elements. The plurality of crimper elements defines a crimper channel. The plurality of crimper elements includes one or more biasing features that are configured to asymmetrically compress the implantable medical device. The crimper also includes handle configured to operate the crimper elements. The movement of the handle displaces the plurality of crimper elements. The displacement of the plurality of crimper elements decreases a volume of the crimper chamber to transition the implantable medical device from the uncompressed arrangement to the compressed arrangement.

In another aspect of the second invention,
the biasing features comprise one or more ridges formed on an interior surface of the plurality of crimper elements.

In another aspect of the second invention,
the biasing features comprise one or more bumps formed on an interior surface of the plurality of crimper elements.

In an example, the present disclosure provides an illustrative method (not claimed) for altering an implantable medical device from an uncompressed arrangement to a compressed arrangement. The illustrative method includes loading a first end of the implantable medical device include a loading system. The loading system includes an inflow loading assembly and an outflow loading assembly and at least one of the inflow loading assembly and the outflow loading assembly includes one or more one or more biasing features that are configured to asymmetrically compress the first end of the implantable medical device. The illustrative method also includes advancing the first end of the implantable medical device into the loading system. A volume of the inflow loading assembly or the outflow loading assembly asymmetrically transitions the first end of the implantable medical device from the uncompressed arrangement to the compressed arrangement.

In an example, the present disclosure provides an illustrative method (not claimed) for altering an implantable medical device from an uncompressed arrangement to a compressed arrangement. The illustrative method includes loading the implantable medical device into a crimper chamber of a crimper. The method further includes actuating a handle of the crimper. The actuation of the handle decreases a volume of the crimper chamber to transition the implantable medical device from the uncompressed arrangement to the compressed arrangement. The crimper chamber includes one or more biasing features that are configured to asymmetrically compress the implantable medical device.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings are not to scale.
FIGS. 1A-1C depict several illustrations of a loading system for use with an implantable medical device, according to an embodiment hereof.
FIGS. 2A-2E depict several illustrations of an inflow loading assembly of the loading system of FIGS. 1A-1C, according to an embodiment hereof.
FIGS. 3A and 3B depict illustrations of another inflow loading assembly of the loading system of FIGS. 1A-1C, according to an embodiment hereof.
FIGS. 4A and 4B depict illustrations of an outflow loading assembly of the loading system of FIGS. 1A-1C, according to an embodiment hereof.
FIGS. 5A-5D depict several illustrations of a capsule guide of the loading system of FIGS. 1A-1C, according to an embodiment hereof.
FIG. 6 depicts an illustration of a tip guide tube of the loading system of FIGS. 1A-1C, according to an embodiment hereof.
FIGS. 7A and 7B depict an illustration of a delivery system that may be used with the loading system of FIGS. 1A-1C, according to an embodiment hereof.
FIGS. 7C and 7D depict an illustration of a prosthetic heart valve that may be used with the loading system of FIGS. 1A-1C, according to an embodiment hereof.
FIG. 8 and FIGS. 9A-9O depict an operation of the loading system of FIGS. 1A-1C, according to an embodiment hereof.
FIGS. 10A and 10B depict a crimper for use with a medical device, according to an embodiment hereof.
FIGS. 11A and 11B depict a crimper element of the crimper of FIGS. 10A and 10B, according to an embodiment hereof.

### DETAILED DESCRIPTION

Specific embodiments of the present disclosure are now described with reference to the figures. The following detailed description describes examples of embodiments and is not intended to limit the present technology or the application and uses of the present technology. Although the description of embodiments hereof is in the context of a loading device, the present technology may also be used in other devices. Furthermore, there is no intention to be bound by any expressed or implied theory presented in the preceding technical field, background, brief summary or the following detailed description.

The terms "distal" and "proximal", when used in the following description to refer to a delivery system or catheter are with respect to a position or direction relative to the treating clinician. Thus, "distal" and "distally" refer to positions distant from, or in a direction away from the treating clinician, and the terms "proximal" and "proximally" refer to positions near, or in a direction toward the clinician.

Embodiments disclosed herein are directed to a loading system for loading an implantable medical device into or onto a delivery device and converting the implantable medical device from an uncompressed (expanded) arrangement to a compressed arrangement. The loading system includes one or more loading assemblies (e.g., inflow loading assemblies and/or outflow loading assemblies) that compress the implantable medical device into a non-circular shape. The loading assemblies induce specific shapes or patterns in structural components of the implantable medical device during loading. These shapes disperse the overlap of the structural components of the implantable medical device into several predetermined locations, reducing the global maximum strain on the structural components of the implantable medical device. As such, the loading system enables implantable medical devices to be loaded into smaller profile delivery systems, without compromising performance of the implantable medical devices. This may allow for an implantable medical device, such as a prosthetic heart valve, to maintain its performance in key areas such as paravalvular leakage, migration resistances, hemodynamics, durability, *etc.*

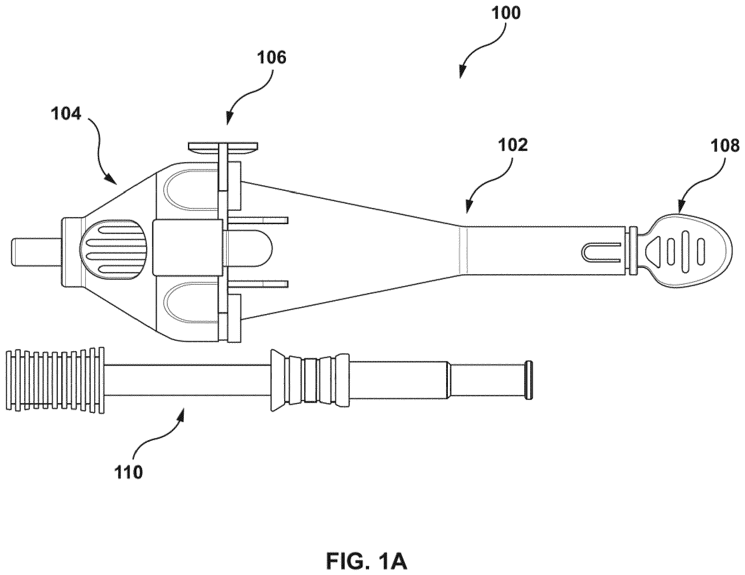

FIGS. 1A-1C illustrate an example of a loading system 100 in accordance with an embodiment hereof. One skilled in the art will realize that FIGS. 1A-1C illustrate one example of a loading system and that existing components illustrated in FIGS. 1A-1C may be removed and/or additional components may be added to the loading system 100.

As disclosed herein, the loading system 100 can be utilized on implantable medical devices (*e.g.,* prosthetic heart valves) that are to be delivered transluminally via portions of a delivery system, *e.g.,* via a catheter, and that need to be loaded onto or into the portions of a delivery system. The loading system 100 can be utilized to radially compress components of the implantable medical device (*e.g.,* stent or frame of a prosthetic heart valve) to have a small profile, *e.g.,* until a diameter of the implantable medical device is as close to the portions of the delivery system as possible. Likewise, during the compression process, the loading system 100 can be utilized to load the implantable medical device (*e.g.,* prosthetic heart valves) into/onto portions of a delivery system such that the implantable medical device can be delivered through the vessels to an implant location in a compressed arrangement, and then expanded at the implant location, for example, by a self-expanding stent/frame or a balloon of the delivery system to replace the native heart valve.

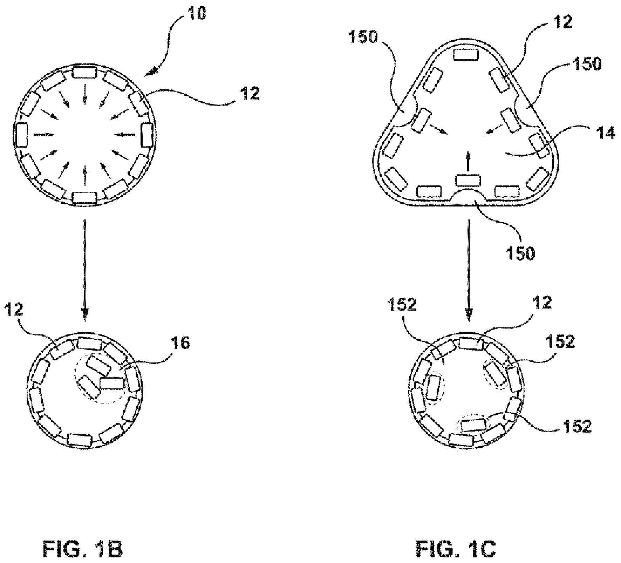

As illustrated in FIG. 1, the loading system 100 includes an inflow loading assembly 102, an outflow loading assembly 104, a backplate 106, a tip guide tube 108, and a capsule guide 110. The inflow loading assembly 102, the outflow loading assembly 104, the backplate 106, the tip guide tube 108, and the capsule guide 110 are configured to operate together to transition an implantable medical device (*e.g.,* prosthetic heart valve) from an uncompressed arrangement to a compressed arrangement. In particular, the inflow loading assembly 102 and the outflow loading assembly 104 includes one or more tapered chambers and/or one or more reduced diameter chambers that apply a compression force on external surfaces of the implantable medical device when introduced to the loading system 100. While transitioning the implantable medical device, the inflow loading assembly 102, the outflow loading assembly 104, the backplate 106, the tip guide tube 108, and the capsule guide 110 are configured to operate together to load the implantable medical device into/onto a delivery system.

Conventional loading systems are typically designed such that the portions of the loading system, which compress the implantable medical device, have a circular cross-section. For example, a conventional loading system may include one or more conical funnels and/or tubes with circular cross-sections that uniformly reduce in diameter to compress the implantable medical device, as illustrated in FIG. 1B, which is a cross-sectional view of a portion of a conventional loading system 10. As illustrated in FIG. 1B, in the case of the loading system 10 with uniform circular reduction in diameter, uniform force is applied to an exterior surface of the implantable medical device. As such, structural components 12 of the implantable medical device (*e.g.,* struts and crowns of a prosthetic heart valve) are compressed symmetrically. The symmetrical compression may cause pressure to build in random locations as all of the structural components 12 of the implantable medical device try to conform to a tight circular shape. The pressure build-up is typically released, unpredictably, by one or more structural components 12 buckling and filling a free space 14 inside the rest of the structural components 12. As illustrated in FIG. 1B, the buckling may cause a concentration 16 of the structural components 12 to form within the free space 14 at an unpredictable location or unpredictable locations. The concentration 16 of the structural components 12 can create elevated crimp strain thereby impacting the structural integrity of the implantable medical device and may produce damage to components of the implantable medical device (*e.g.,* valve leaflets in a prosthetic heart valve.)

In embodiments, to address these drawbacks and allow loading in low profile delivery systems, the inflow loading assembly 102 and/or the outflow loading assembly 104 are designed to bias select portions of the implantable medical device towards a central axis of the implantable medical device. In some embodiments, the inflow loading assembly 102 and/or the outflow loading assembly 104 include one or more portions that have a non-circular cross-sections to compress the implantable medical device, as described below in further detail. In some embodiments, the inflow loading assembly 102 and/or the outflow loading assembly 104 include one or more biasing features 150 that apply a compression force, unevenly, to the exterior surfaces of the implantable medical device, as illustrated in FIG. 1C and discussed below in further detail. The biasing features 150 are designed to cause overlap of the structural components 12 of the implantable medical device at multiple and select locations. As such, the biasing features 150 distribute the overlap of the structural components evenly within the free space 14 of the implantable medical device, thereby reducing the occurrence of a concentration of the structural components of the implantable medical device. The distributed overlap may allow for safe loading the implantable medical device into lower profile delivery systems. For example, as illustrated in FIG. 1C, in the case of a non-uniform loading system, the structural components 12 are compressed asymmetrically. This prevents circumferential pressure build-up by biasing the structural components inwards in predefined areas. This gives predictability in where an overlap 152 of the structural components may occur.

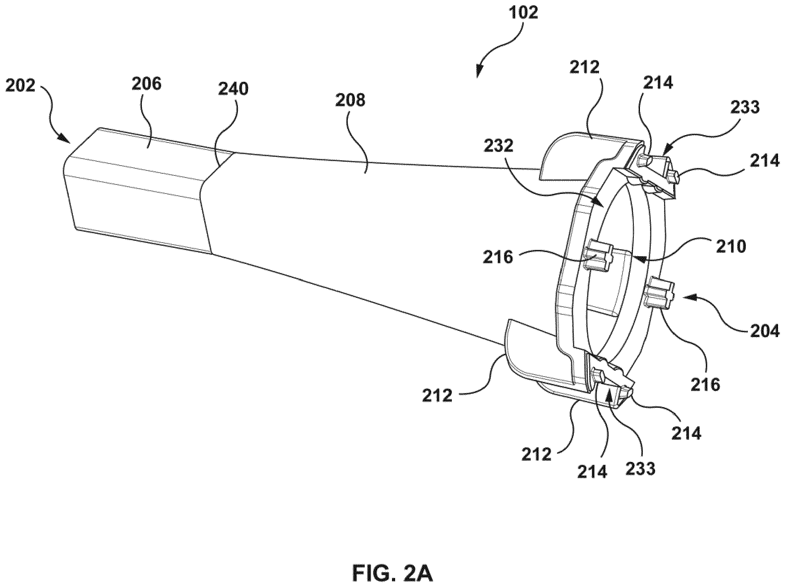

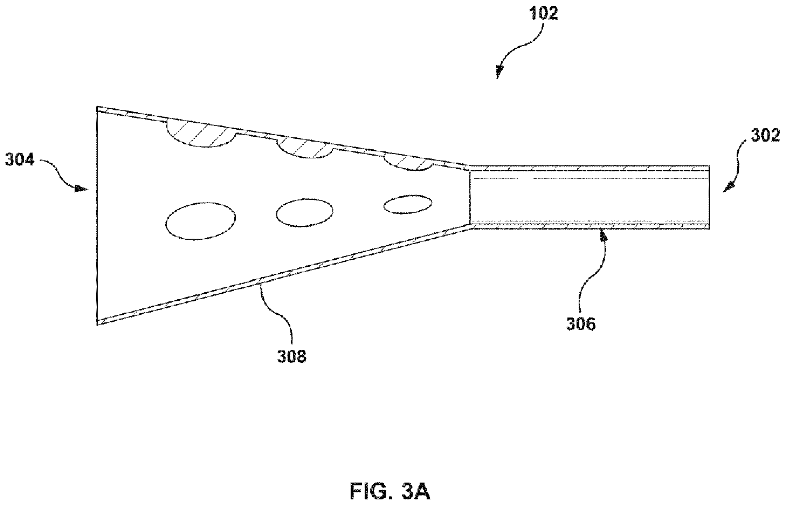

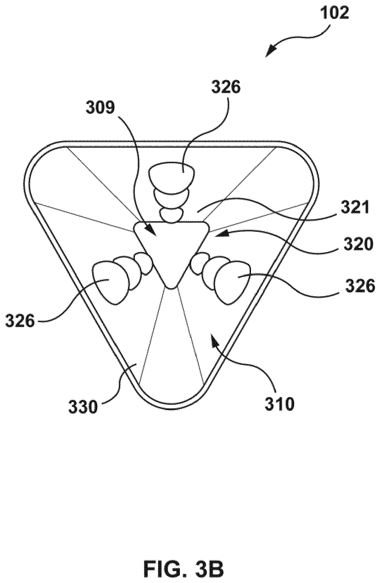

Returning to FIG. 1A, in embodiments, the outflow loading assembly 104 is configured to couple with the inflow loading assembly 102. The inflow loading assembly 102 is configured to hold the implantable medical device such that one end of the implantable medical device, *e.g.,* an outflow portion of a prosthetic heart valve, can be compressed by the outflow loading assembly 104 and the capsule guide 110. Additionally, or alternatively, the inflow loading assembly 102 is configured to crimp the other end of the medical device, *e.g.,* an inflow portion of a prosthetic heart valve, and to hold the medical device at a compressed arrangement until the implantable medical device is loaded into/onto the delivery device. One example of the inflow loading assembly 102 is described in further detail below with reference to FIGS. 2A-2E. Additionally, another example of the inflow loading assembly 102 is described in further detail below with reference to FIGS. 3A and 3B.

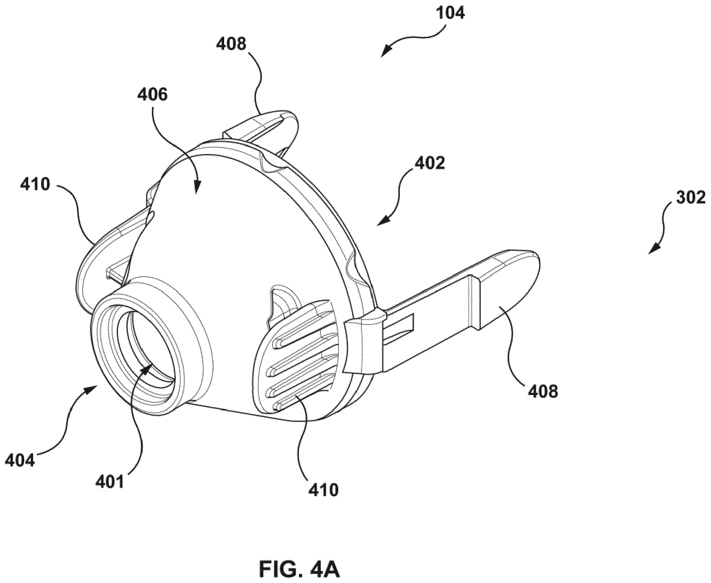

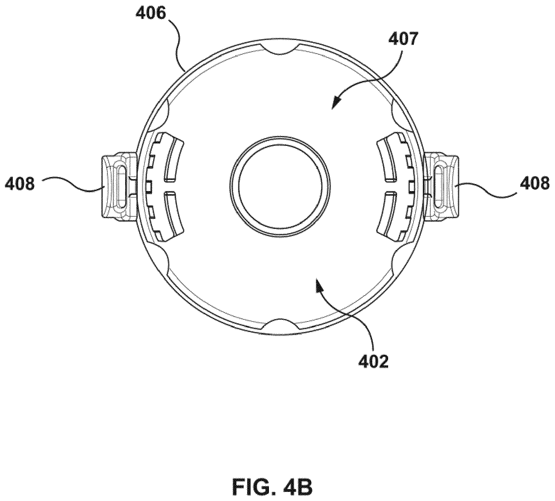

In embodiments, the outflow loading assembly 104 is configured to partially compress one end of implantable medical device, for example, an outflow end of a prosthetic heart valve. Additionally, or alternatively, the outflow loading assembly 104 is configured to operate in combination with the capsule guide 110 to compress one end of the implantable medical device and load the implantable medical device onto the delivery system. One example of the outflow loading assembly 104 is described in further detail below with reference to FIGS. 4A and 4B.

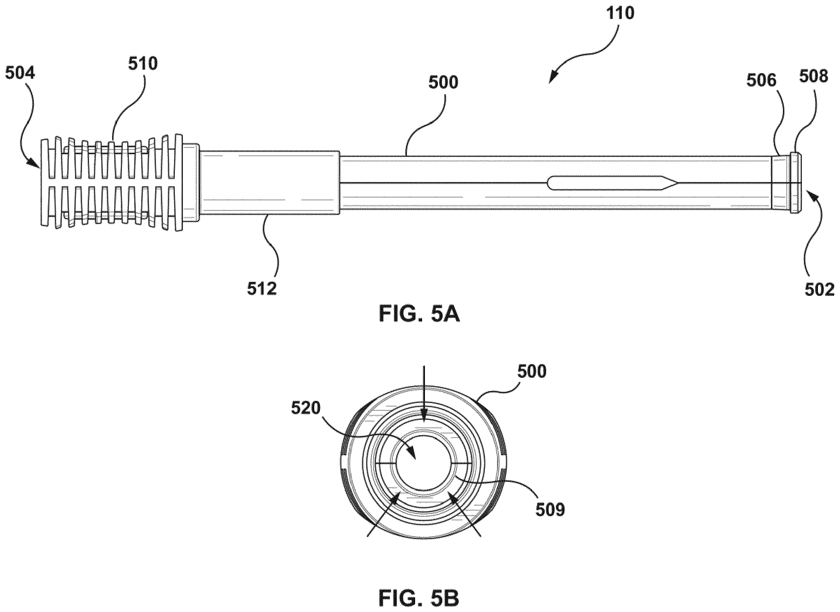

In embodiments, the capsule guide 110 is configured to provide additional column support for protecting a distal portion of a delivery device, for example, a capsule of a delivery catheter, during loading. Additionally, or alternatively, the capsule guide 110 is configured to notify a user of a potential misload. The capsule guide 110 may also be configured to interface the coupling members of an implantable medical device, for example, paddles of a prosthetic heart valve, with the coupling members of the attachment member of the delivery device, for example, recesses in a spindle of a delivery catheter. The capsule guide 110 may be also configured to allow for inspection by a user that correct coupling has occurred, for example, that the paddles are correctly seated within the recess. One example of the capsule guide 110 is described in further detail below with reference to FIGS. 5A-5D.

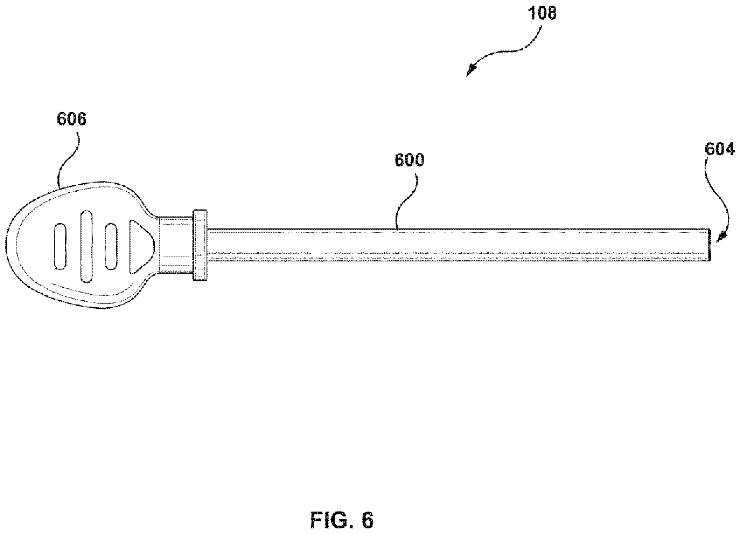

In embodiments, the tip guide tube 108 is configured to allow a tip of a delivery device to pass atraumatically through the implantable medical device and to spread open one end of the medical device, *e.g.,* the outflow end of a prosthetic heart valve including outflow crowns and paddles, to align the coupling members of the implantable medical device with the coupling members of the attachment member of the delivery device as described below. One example of the tip guide tube 108 is described in further detail below with reference to FIG. 6.

In some embodiments, at least one portion of one or more components of the loading system 100 can be transparent. For example, the inflow loading assembly 102, the outflow loading assembly 104, the backplate 106, the tip guide tube 108, and the capsule guide 110 can each be transparent. This transparency allows a user to visually verify the proper orientation and coupling of an implantable medical device being loaded as further described below. Components of the loading system can made of any suitable material or materials. For example, the inflow loading assembly 102, the outflow loading assembly 104, the backplate 106, the tip guide tube 108, and the capsule guide 110 can be made of materials commonly used in medical device applications such as suitable polymeric materials, metals, and the like.

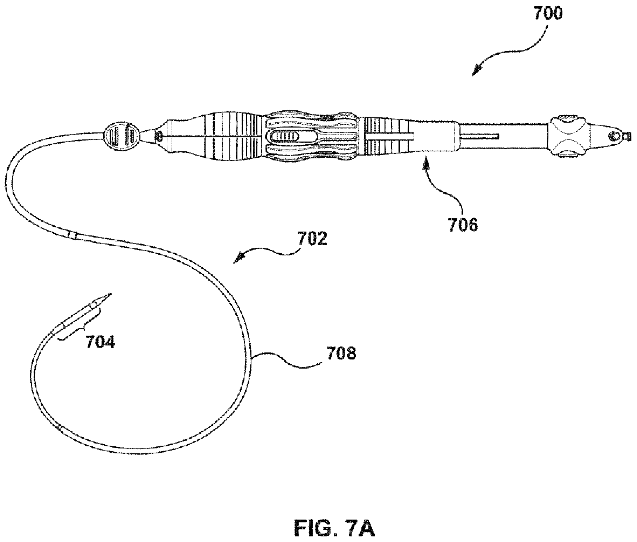

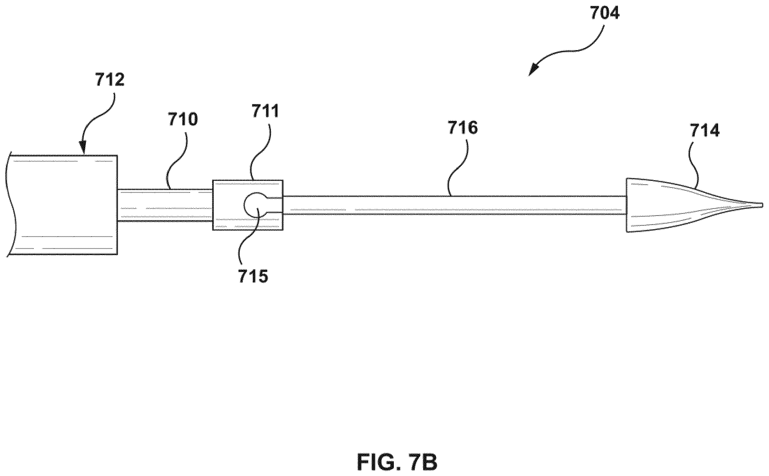

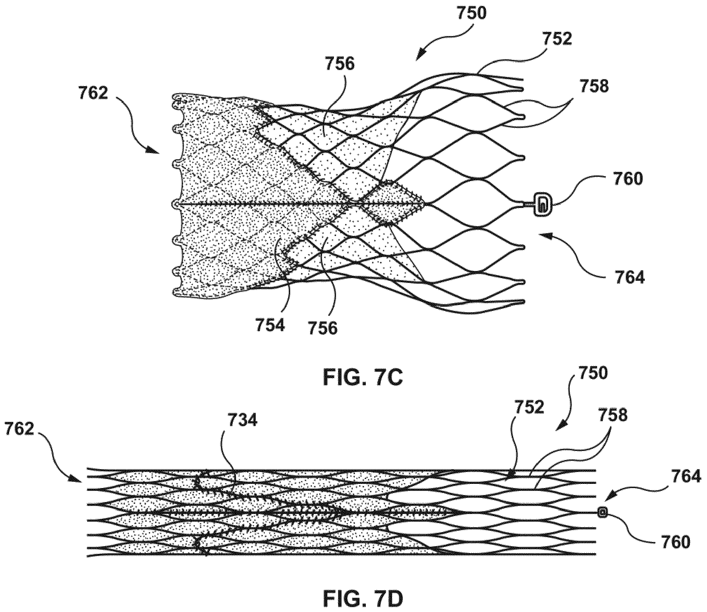

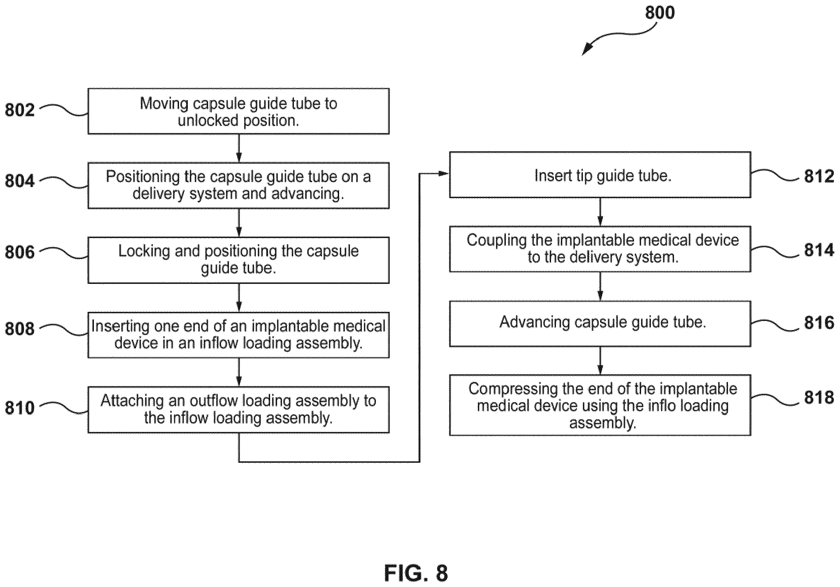

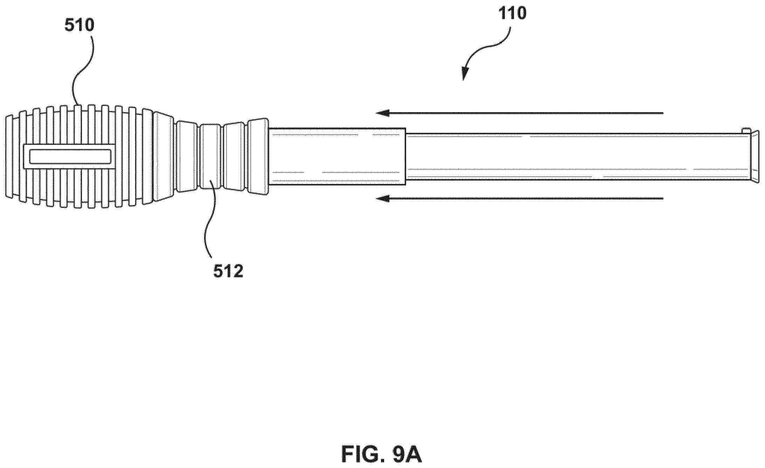

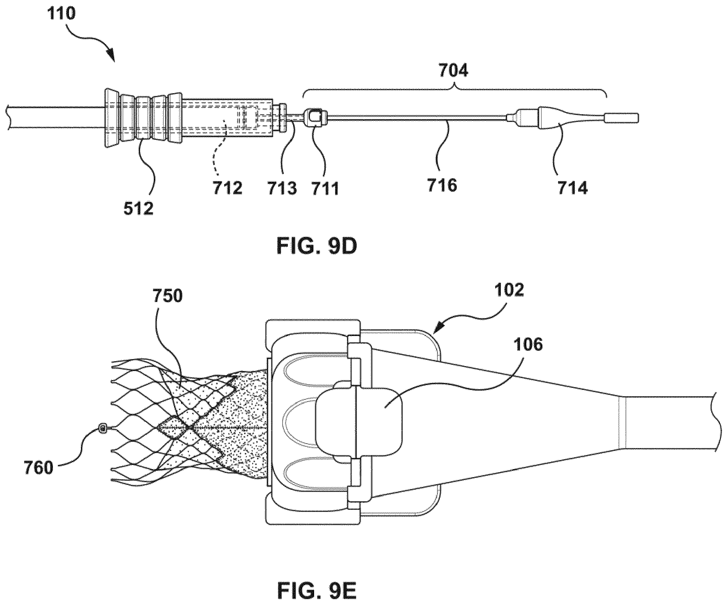

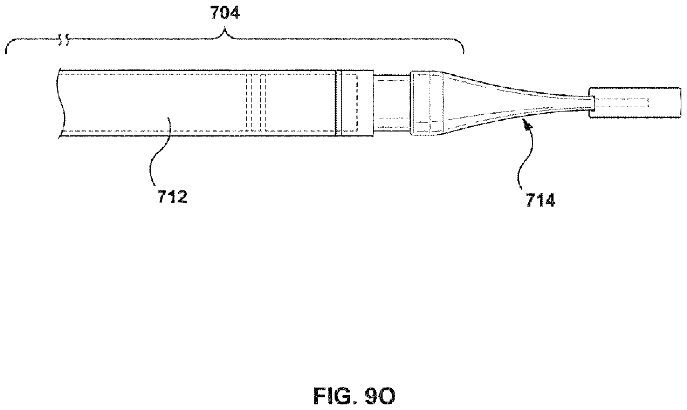

The loading system 100 is configured to convert an implantable medical device from its uncompressed arrangement to its compressed arrangement and to load the implantable medical device into/onto portions of a delivery system, as described below in further detail with reference to FIGS. 8 and 9A-O. One example of a delivery system is described in further detail below with reference to FIGS. 7A and 7B. One example of implantable medical device is described in further detail below with reference to FIGS. 7B and 7C.

FIGS. 2A-2E illustrate an example of the inflow loading assembly 102 in accordance with an embodiment hereof. One skilled in the art will realize that FIGS. 2A-2E illustrate one example of an inflow loading assembly and that existing components illustrated in FIGS. 2A-2E may be removed and/or additional components may be added to the inflow loading assembly 102.

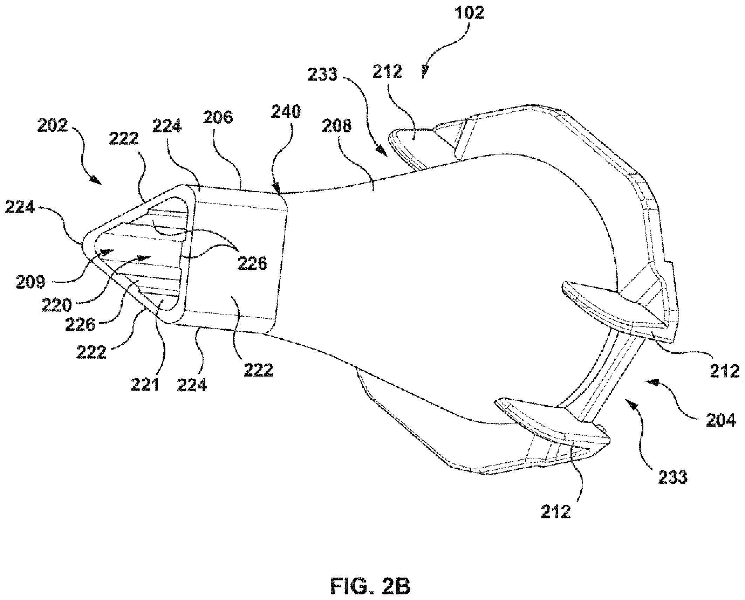

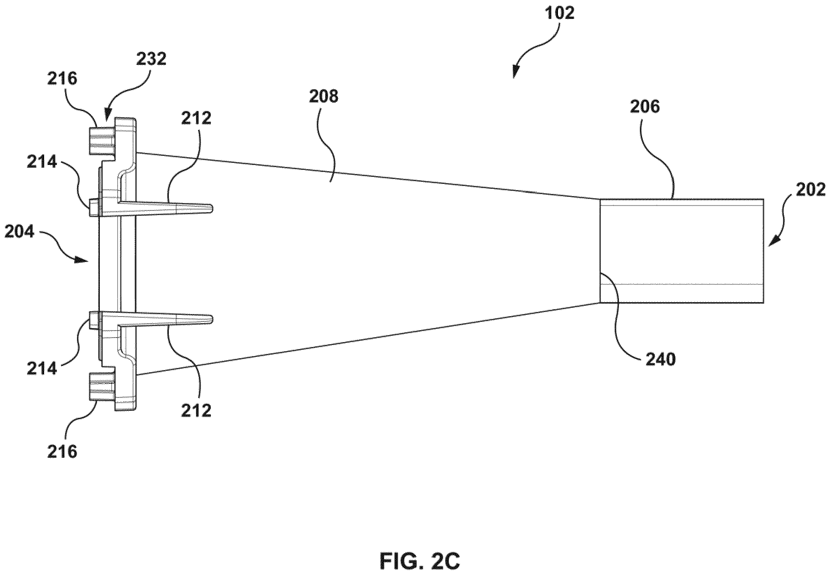

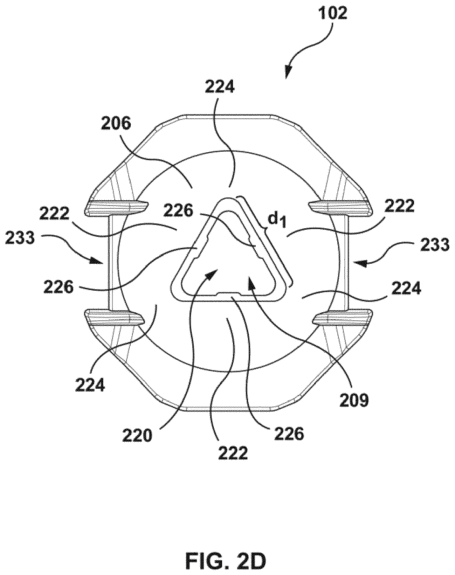

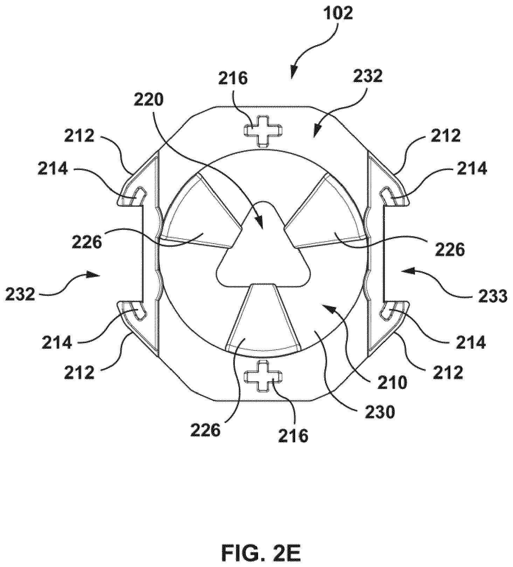

As illustrated in FIG. 2A and 2B, which are perspective views of the inflow loading assembly 102 and FIG. 2C, which is a side view, the inflow loading assembly 102 includes a distal end 202 and a proximal end 204. The inflow loading assembly 102
includes a first potion 206 and a second portion 208. As illustrated in FIGS. 2D and 2E, which are axial views of the distal end 202 and the proximal end 204, respectively, the inflow loading assembly 102 may define a channel 220 extending from the distal end 202 to the proximal end 204, thereby forming a distal opening 209 and a proximal opening 210, respectively. The channel 220 of the inflow loading assembly 102 may be configured to receive and to compress or partially compress one end of an implantable medical device. In some embodiments, as described below, the configuration of the channel reduces potential damage to the implantable medical device during compression. When the inflow loading assembly 102 is coupled to the outflow loading assembly 104, the channel 220 extending from the distal end 202 to the proximal end 204 is coaxial with a channel extending from respective ends of the outflow loading assembly 104 (e.g., ends 402, 404, and channel 420 described below with reference to FIGS. 4A and 4B). An inner dimension (e.g., cross-sectional area) of the proximal opening 210 is larger than an inner dimension (e.g., cross-sectional area) of the distal opening 209.

The second portion 208, including the proximal opening 210, is configured to secure, to guide, and to position one end of an implantable medical device, e.g., an inflow end of a prosthetic heart valve, by an interference fit as described below referring to FIGS. 8 and 9A-9O. For example, beginning at the proximal opening 210 at the proximal end 204, the second portion 208 can have a tapered or modified geometry that operates to compress a portion of the implantable medical device when advanced from the proximal opening 210 into the second portion 208. In embodiments, the second portion 208 of the inflow loading assembly 102 is configured to compress one end of an implantable medical device, for example, the inflow portion of a prosthetic heart valve, as the implantable medical device slides against an inner surface 230 of the second portion 208. The inner surface 230 decreases in internal diameter in a direction from the proximal end 204 to the distal end 202. In some embodiments, the second portion 208 can be formed having a frustoconical inner surface. In some embodiments, the second portion 208 has a curved or stepped inner surface that tapers. Furthermore, although the inner surface 230 of the second portion 208 of the inflow loading assembly 102 is generally circular in cross-sectional shape as shown in FIG. 2E, other suitable shapes may be employed, as described below in further detail with reference to FIGS. 3A and 3B. Additionally, although an outer surface of the second portion 208 has a shape that generally corresponds to the inner surface 230 of the second portion 208 in FIGS. 2A and 2B, in some embodiments, the outer surface can be formed in a shape that differs from the inner surface 230 of the second portion 208 and can have any suitable shape.

While FIGS. 2A-2E illustrate the second portion 208 having a circular cross-section, the second portion 208 can be formed to have any non-circular cross-section that provides a non-uniform compression force. For example, the second portion 208 can be formed in a regular three-dimensional shape, such as a hollow geometric prism with four or more sides. Likewise, for example, the second portion 208 can be formed in an irregular three-dimensional shape.

The second portion 208 of the inflow loading assembly 102 also defines a slot 232 in communication with the channel 220 extending between the distal end 202 and the proximal end 204. The slot 232 may be positioned at the proximal end 204 and configured to slidably receive the backplate 106. In embodiments, the slot 232 can be defined by the tabs 212 positioned at opposing sides of the proximal opening 210. A size and shape of slot 232 substantially corresponds to a cross-sectional shape of backplate 106. For example, as shown in FIG. 2E, the slot 232 can be approximately rectangular, which corresponds to the rectangular cross-sectional shape of the backplate 106, and can include one or more stops 216 for positioning the backplate 106. In embodiments, the backplate 106 can operate as a stop for one end of the implantable medical device. The second portion 208 also includes tabs 212 that define engagement slots 233. The tabs 212 are configured to engage with engagement tabs of the outflow loading assembly 104, described below with reference to FIGS. 4A and 4B. That is, the engagement tabs of the outflow loading assembly 104 are aligned with the tabs 212 of the inflow loading assembly 102, and the outflow loading assembly 104 is advanced until the engagement tabs of the outflow loading assembly 104 enter the engagement slots 233 and engage the tabs 212. In some embodiments, the tabs 212 can include one or more pins 214 that can engage with the outflow loading assembly 104.

The first portion 206 of the inflow loading assembly 102 is configured to hold an implantable medical device at a compressed arrangement until the implantable medical device is loaded on a delivery device, for example, within a capsule of a delivery catheter. In some embodiments, the first portion 206 has an inner surface 221 having a circular cross-section. In some embodiments, as illustrated in FIG. 2D and discussed below in further detail, the first portion 206 can have a non-cylindrical inner surface 221 having a non-circular cross-section. In some embodiments, the first portion 206 can have a tapered inner surface that reduces in diameter.

As illustrated in FIG. 2A, the first portion 206 is adjacent and distal to the second portion 208 and are coupled at a junction 240. In some embodiments, an inner dimension (e.g., cross-sectional surface area) of the first portion 206 is smaller than an inner dimension (e.g., cross-sectional surface area) of the junction 240 of the second portion 208 and the first portion 206. In some embodiments, an inner dimension of the first portion 206 is sized such that the tip guide tube 108 can pass through an implantable medical device compressed and loaded within the first portion 206 and such that the implantable medical device is compressed as much as possible before being loaded within a portion of a delivery system, e.g., withdrawn into a delivery portion of a catheter. In some embodiments, an axial length of the first portion 206 is substantially equal to or greater than an axial length of the medical device. In some embodiments, the first portion 206 is separate from the second portion 208 and the first portion 206 is coupled to the second portion 208, for example, by ultrasonic welding or a snap fit. In some embodiments, the first portion 206 is integral with the second portion 208.

As illustrated in FIGS. 2B and 2D, the inner surface 221 of the first portion 206 can be formed to have an approximate triangular shape formed by sidewalls 222 coupled at corners 224, thereby defining the distal opening 209 with an approximately triangular shape. That is, the portion of the channel 220 formed by the first portion 206 can be formed in the three-dimensional shape of a hollow geometric prism with three sides. The triangular cross-sectional shape of the inner surface 221 of the first portion 206 is configured to provide an asymmetric compression force on the outer surfaces of the implantable medical device. For example, the implantable medical device may generally have a tubular shape with a circular or elliptical cross-section, for example, as illustrated in FIGS. 7B and 7C. Accordingly, when the tubular-shaped implantable medical device is introduced to the portion of the channel 220 formed by the first portion 206, the triangular cross-sectional shape of the inner surface 221 of the first portion 206 provides a compression force that is non-uniform on the exterior surfaces of the tubular-shaped implantable medical device, as discussed above with reference to FIG. 1C. Although an outer surface of the first portion 206 has a shape that generally corresponds to the inner surface 221 of the first portion 206 in FIGS. 2A, 2B, and 2D, in some embodiments, the outer surface can be formed in a shape that differs from the inner surface 221 of the first portion 206 and can have any suitable shape.

While FIGS. 2A-2E illustrate the first portion 206 having a triangular cross-section, the first portion 206 can be formed to have any non-circular cross-section that provides a non-uniform compression force. For example, the first portion 206 can be formed in a regular three-dimensional shape, such as a hollow geometric prism with four or more sides. Likewise, for example, the first portion 206 can be formed in an irregular three-dimensional shape.

In embodiments, the proximal opening 206 is formed in an approximate equilateral triangular cross-section formed by sidewalls 222 that are coupled at corners 224. Each sidewall 222 can be formed to a length, *d₁.* The sidewalls 222 are formed with the length, *d₁* so that the tip guide tube 108 can pass through an implantable medical device compressed and loaded within the first portion 206 and such that the implantable medical device is compressed as much as possible before being loaded with a portion of a delivery system, e.g., withdrawn into a delivery portion of a catheter. In embodiments, the length, *d₁,* of the sidewalls 222 may depend on the French (FR) size of the catheter or the size of the implantable medical device. For example, the length, *d₁,* of the sidewalls 222 may be formed to accommodate a 18-33 Fr catheter and/or a 23-24 mm implantable medical device.

As illustrated in FIGS. 2A and 2B, the proximal opening 210 of the second portion 208 can be formed having a diameter, *f₁*. The junction 240 of the first portion 206 and the second portion 208 can be formed having a diameter, *f₂*. The inner surface 230of the second portion 208 forms a compression chamber defined by the decreasing diameter of the second portion 208 from the proximal opening 210 (the diameter,*f₁*) and to the junction 240 (the diameter, *f₂*). In some embodiments, the decreasing diameter of the compression chamber of the second portion 208 can be formed in an approximate funnel or cone shape. In embodiments, a rate or degree at which the diameter decreases from the proximal opening 210 to the junction 240, *e.g.,* slope, can affect the angle at which the implant attachment tabs exit the second portion 208, with a longer taper improving the loading of an implantable medical device. The longer taper may provide a smoother transition for the implantable medical device during loading into the delivery device. In embodiments, the diameter decreases from the proximal opening 210 to the junction 240 and operates to apply a compression force on the implantable medical device as the implantable medical device is moved through the compression volume. In embodiments, the diameter, *f₂* at the junction 240 of the second portion 208 and the first portion 206 may depend on the FR size of the catheter. For example, the diameter, *f₂*, of the second portion 208 may be formed to accommodate a 18-33 Fr catheter. In embodiments, the diameter, *f₁*, of the proximal opening 210 of the second portion 208 may depend on an outer diameter of the implantable medical device.

In embodiments, as illustrated in FIGS. 2A-2E, the first portion 206 and the second portion 208 can be formed having different cross-sectional shapes thereby defining the channel 220 having a different shape for the first portion 206 and a different shape for the second portion 208. That is, the first portion 206 can have a triangular cross-section and the second portion 208 can have a circular cross-section.

Interior surfaces of the inflow loading assembly 102 include one or more biasing features that provide an asymmetric compression force. For example, as illustrated in FIGS. 2D and 2E, the channel 220 can include three biasing features 226. Each of the biasing features 226 can be formed as a rectangular ridge that extends on the inner surfaces of the first portion 206 and the second portion 208 from the distal opening 209 to the proximal opening 210. In embodiments, when formed as a rectangular ridge, each of the biasing features 226 can be formed to any dimensions, *e.g.,* width, depth, length, in order to provide an asymmetric compression force on the implantable medical device. In some embodiments, a width of a biasing feature 226 can range from approximately 1 millimeter (mm) to approximately 20 mm. In some embodiments, a length of a biasing feature 226 can range from approximately 10 mm to approximately 40 mm. In some embodiments, the dimensions of the biasing features 226 can be constant. In some embodiments, the dimensions of the biasing features 226 can vary. For example, the width and/or the depth of biasing features 226 can increase and/or decrease along the length of the biasing features 226. That is, each of the biasing features 226 may have a larger width and/or the depth at the proximal opening 210 as compared to the distal opening 209, or *vice versa.* While FIGS. 2A-2E illustrate biasing feature 226 as being rectangular ridge, one skilled in the art will realize that a biasing feature can be formed in any shape and/or size.

In embodiments, as illustrated in FIGS. 2A-2E, the biasing features 226 can be formed on interior surfaces of the first portion 206 and the second portion 208. Likewise, in some embodiments, the biasing features 226 can be formed on only the interior surfaces the first portion 206 or the interior surfaces of the second portion 208.

FIGS. 3A and 3B illustrate simplified views of another example of the inflow loading assembly 102. As illustrated in FIG. 3A, which is a side view, the inflow loading assembly 102 includes a distal end 302 and a proximal end 304. The inflow loading assembly 102 includes a first portion 306 and a second portion 308. As illustrated in FIG. 3B, which is a view of the proximal end 304, the first portion 306 and the second portion 308 define a channel 320 that extends from a proximal opening 310 to a distal opening 309. As illustrated, the first portion 306 and the second portion 308 can be formed having a same cross-sectional shape, *e.g.,* a hollow geometric prism with three sides. For example, the first portion 306 and the second portion 308 can have an approximate triangular shape formed by the inner surface 321 of the first portion 306 and the inner surface 330 of the second portion 308, thereby defining the distal opening 309 and the proximal opening 310 with an approximate triangular shape. The triangular cross-sectional shape of inner surfaces of the first portion 306 and the second portion 308 are configured to provide an asymmetric compression force on the outer surfaces of the implantable medical device.

In embodiments, as illustrated in FIGS. 3A and 3B, the biasing features 326 can be formed as semi-circular bumps. The semi-circular bumps can be formed on the inner surface 321 of the first portion 306 and the inner surface 330 of the second portion 308. In embodiments, when formed as semi-circular bumps, each of the biasing features 326 can be formed to any dimensions, *e.g.,* radius, in order to provide an asymmetric compression force on the implantable medical device. In some embodiments, a radius of a biasing feature 326 can range from approximately 1 mm to approximately 20 mm. In some embodiments, the biasing features 326 can be formed as oval shaped bumps. In this embodiment, a width of the oval shape can range from approximately 1 mm to approximately 13 mm, and a length of the oval shape can range from approximately 10 mm to approximately 20 mm. In some embodiments, the dimensions of the biasing features 326 can be constant. In some embodiments, the dimensions of the biasing features 326 can vary. For example, the radius of biasing features 326 can increase and/or decrease along the length of the inflow loading assembly 102. That is, each of the biasing features 326 may have a larger radius at the proximal opening 310 as compared to the distal opening 309, or *vice versa.* While FIGS. 3A and 3B illustrate biasing feature 326 as being discrete semi-circular bumps, one skilled in the art will realize that a biasing feature can be formed in any shape and/or size.

In embodiments, the biasing features 326, as illustrated in FIGS. 3A and 3B, can be formed on the inner surface 321 of the first portion 206 and/or the inner surface 330 the second portion 308. Likewise, in some embodiments, the biasing features 326 can be formed on only the interior surfaces the first portion 306 or the inner surface 330 of the second portion 208, as illustrated FIG. 3B. Likewise, while FIGS. 3A and 3B illustrate the inflow loading assembly 102 including nine (9) biasing features 326, three (3) on each side channel 320 in the second portion 308, one skilled in the art will realize that the inflow loading assembly 102 can include any number of biasing features 326.

Further, as illustrated in FIGS. 3A and 3B, the first portion 306 and the second portion 308 can be formed having a same cross-sectional shape, *e.g.,* a triangular shape. While FIGS. 3A and 3B illustrate the first portion 306 and the second portion 308 having a triangular cross-section, the first portion 306 and the second portion 308 can be formed to have any non-circular cross-section that provides an asymmetric compression force. For example, the first portion 306 and the second portion 308 can be formed in a regular three-dimensional shape, such as a hollow geometric prism with four or more sides. Likewise, for example, the first portion 306 can be formed in an irregular three-dimensional shape.

FIGS. 4A and 4B illustrate an example of the outflow loading assembly 104 in accordance with an embodiment hereof. One skilled in the art will realize that FIGS. 4A and 4B illustrate one example of an outflow loading assembly and that existing components illustrated in FIGS. 4A and 4B may be removed and/or additional components may be added to the outflow loading assembly 104.

As illustrated in FIG. 4A, which is a perspective view, the outflow loading assembly 104 defines a channel 401 extending from a distal open end 402 to a proximal open end 404. The outflow loading assembly 104 may include a portion 406 having a tapered inner surface 407 that has an inner dimension that decreases, as illustrated in FIG. 4B, which is a view from the distal open end 402. In some embodiments, the tapered inner surface 407 continuously decreases, from the distal open end 402 to the proximal open end 404. In embodiments, the inner dimension (*e.g.,* cross-sectional area) of the proximal open end 404 is smaller than the inner dimension (*e.g.,* cross-sectional area) of the distal open end 402. The inner dimension of the proximal open end 404 is preferably sized to allow the capsule guide 110 to pass therethrough. The inner dimension of the distal open end 402 is preferably sized to receive an end of the implantable medical device, for example, an outflow end of a prosthetic heart valve, and compress the end the implantable medical device as the outflow loading assembly 104 is moved towards the inflow loading assembly 102. The inner dimension of the distal open end 402 is preferably sufficient to encompass the end of the implantable medical device without damaging the implantable medical device. The angle of the tapered surface relative 107 to the longitudinal axis of the outflow loading assembly 104, the inner diameter of the proximal open end 404, and the length between the portion 406 and the proximal open end 404 may vary dependent on the size or design of the medical device to ensure a consistent interface with the delivery system.

In some embodiments, the portion 406 has a frustoconical inner surface. In some embodiments, the portion 406 has a curved or stepped inner surface that tapers. Furthermore, although the portion 406 of the outflow loading assembly 104 is generally circular in cross-section, other suitable shapes that load the medical device without damage may be employed. Additionally, although the outer surface of the portion 406 has a shape that generally corresponds to the inner surface of the portion 406, as in FIGS. 4A and 4B, in some embodiments, the outer surface does not corresponded to the tapered inner surface of the portion 406.

The outflow loading assembly 104 is configured to couple with the inflow loading assembly 102. For example, in some embodiments, the outflow loading assembly 104 includes one or more engagement tabs 408 configured to selectively couple to the inflow loading assembly 108, for example, by coupling to respective tabs 412 defined by outflow loading assembly 102, as described above with reference to FIGS. 2A-2E. As shown in FIG. 4A, the outflow loading assembly 104 can include an opposing pair of the engagement tabs 408 extending in a distal direction from the distal open end 402. In some embodiments, the outflow loading assembly 104 can include one tab 408 or more than two tabs 408.

The outflow loading assembly 104 can be ergonomically designed to facilitate easy handling by a user. For example, as shown in FIG. 4A, the outflow loading assembly 104 can include one or more gripping tabs 410. The gripping tabs 410 protrude from the exterior surface of the portion 406. A user can easily place a thumb and index finger on the gripping tabs 410 to handle the outflow loading assembly 104.

FIGS. 5A-5D illustrate an example of the capsule guide 110 in accordance with an embodiment hereof. One skilled in the art will realize that FIGS. 5A-5D illustrate one example of a capsule guide and that existing components illustrated in FIGS. 5A-5D may be removed and/or additional components may be added to the capsule guide 110.

As illustrated in FIG. 5A, which is a side view, the capsule guide 110 includes a main body portion 500 having a distal open end 502 and a proximal open end 504. As illustrated in FIG. 5B, which is an axial view of the distal open end 502, the main body portion 500 preferably defines a channel having a distal open end 502 and a proximal open end 504. In some embodiments, the main body portion 500 has a substantially cylindrical outer surface. The main body portion 500 can protect a delivery portion (*e.g.,* capsule) of the delivery device by reducing or preventing the capsule from excessively bowing or being pinched by the user via additional column support. The capsule guide 110 can also comprise a tip 506. In some embodiments, the tip 506 can be elastomeric. In some embodiments, the tip 506 has a tapered outer surface, where an outer dimension of the tip 506 decrease in a direction from the proximal open end 504 to the distal open end 502. The outer dimension of main body portion 500 and tip 506 is preferably smaller than an inner dimension of the distal open end 402 and the proximal open end 404 of the outflow loading assembly 104, and/or smaller than an inner dimension of the proximal end 204 and the second portion 208 of the inflow loading assembly 102, so the main body portion 500 and the tip 506 can preferably pass into the channel collectively defined by the outflow loading assembly 104 and the inflow loading assembly 102. In some embodiments, an inner dimension of the tip 506 is smaller than an outer dimension of a tip of a delivery device.

The exterior surface of the main body portion 500 defines an exterior shoulder 508 that extends radially outward at the distal open end 502. As illustrated in FIG. 5B, the interior surface of the main body portion 500 may define an interior shoulder 509 that extends radially inward adjacent to the tip 506. The interior shoulder 509 can be sized to prevent a capsule of a delivery device from distally advancing past the interior shoulder 509 and through distal open end 502 and into tip 506. In some embodiments, a portion of main body portion 500 adjacent and proximal to interior shoulder 509 is configured to prevent the capsule of a delivery system from expanding during loading. In some embodiments, this portion of main body portion 500 adjacent interior shoulder 509 is a tight tolerance area that provides a tight fit with the capsule of the delivery system and/or substantially prevents the capsule from expanding during loading. In some embodiments, the inner dimension of a portion of main body portion 500 adjacent interior shoulder 509 is sized such that if there is a misload between the delivery catheter and the medical device, a noticeable increase in the amount of force required to load the medical device within the capsule will occur because the outer dimension of the medical device will be larger than the inner dimension of the portion of main body portion 500 adjacent interior shoulder 509.

The capsule guide 110 can include, in some embodiments, a handle portion 510. The handle portion 510 can be ergonomically designed to facilitate easy handling of the capsule guide 110. In some embodiments, the handle portion 510 extends radially outward from the main body portion 500. For example, as shown in FIG. 5A, the handle portion 510 can have a shape that allows handling by a user. The main body portion 500 can have an axial length such that when the handle portion 510 abuts proximal open end 404 of the outflow loading assembly 104, the open end 502 is adjacent a proximal end 204 of the first portion 206 of the inflow loading assembly 102.

In some embodiments, the handle portion 510 has an outside diameter that is larger than an inside diameter of the proximal open end 404 of the outflow loading assembly 104. In such embodiments, the handle portion 510 can function as a stop preventing further distal movement of the capsule guide 110 relative to the outflow loading assembly 104. The capsule guide 110 can include a locking collar 512 slidably coupled to the exterior surface of the main body portion 500. The locking collar 512 is preferably configured to slide axially from the handle 510 to the tip 506. In some embodiments, as illustrated in FIG. 1A, the locking collar 512 can be ergonomically designed to facilitate easy sliding of the locking collar 512. In some embodiments, the main body portion 510 can be formed as separate halves. In this embodiment, the locking collar 512 can compress the two halves of the capsule guide 110 together. The compression of the capsule guide 110 can form a ring that limits a flare of the delivery system from expanding. Additionally, or alternatively, the compression of the locking collar 512 can protect components the delivery system and/or the capsule guide 110 from damage as the prosthetic heart valve 750 is being loaded. As such, the locking collar 512 can cause the capsule guide 110 and a flexible capsule to operate as rigid objects, while the locking collar 512 is engaged.

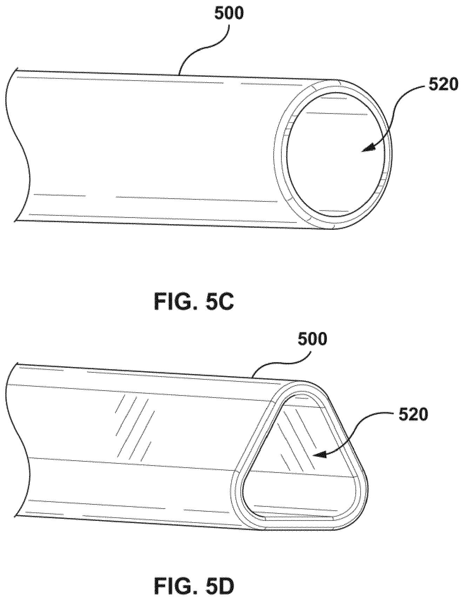

As illustrated in FIGS. 5B and 5C, the main body portion 500 can include a substantially cylindrical inner surface that defines a channel 520 having a circular cross-section. In other embodiments, as illustrated in FIG. 5D, the main body portion 500 can include a substantially triangular inner surface that defines the channel 520 having a triangular cross-section. For example, the cross-sectional shape of the channel 520 can match the cross-section shape of the first portion 206 and/or the section portion 208 of the inflow loading assembly.

FIG. 6 illustrates an example of the tip guide tube 108 in accordance with an embodiment hereof. One skilled in the art will realize that FIG. 6 illustrates one example of a tip guide tube and that existing components illustrated in FIG. 6 may be removed and/or additional components may be added to the tip guide tube 108.

The tip guide tube 108 can include a main body portion 600. The main body portion 600 preferably isolates and protects an implantable medical device, for example, the valve material of a prosthetic valve, from a delivery system passing through the implantable medical device. The main body portion 600 may define a channel having an open end 604. In some embodiments, the main body portion 600 has a substantially cylindrical outer surface. An outer diameter of the main body portion 600 is smaller than an inner dimension of distal open end 402 and second proximal open end 404 of the outflow loading assembly 104, and/or smaller than an inner dimension of the proximal end 204 and the distal end 202 of the inflow the inflow loading assembly 102, so the main body portion 600 can preferably pass through the channel collectively defined by the outflow loading assembly 104 and the inflow the inflow loading assembly 102 (and a slot defined by the backplate 106).

The tip guide tube 108 can include, in some embodiments, a handle portion 606. The handle portion 606 can be ergonomically designed to facilitate easy handling by a user. For example, as shown in FIG. 6, the handle portion 606 can have a substantially flat paddle shape. The main body portion 600 has an axial length such that, when the outflow loading assembly 104 is coupled to the inflow loading assembly 102 and the main body portion 600 passes through the channel collectively defined by the outflow loading assembly 104 and the inflow loading assembly 102, the open end 604 extends beyond the proximal open end 404 of the outflow loading assembly 104 and/or the handle portion 606 extends beyond the distal end 202 of the inflow loading assembly 102.

In some embodiments, the handle portion 606 has an outer dimension that is larger than an inner dimension of the distal end 202. In such embodiments, the handle portion 606 can function as a stop preventing further proximal movement of the tip guide tube 108 relative to the inflow loading assembly 102. In some embodiments, when the handle portion 606 abuts the distal end 202 of the inflow loading assembly 102, the open end 604 of the tip guide tube 108 extends beyond the second proximal open end 404 of the outflow loading assembly 104. In some embodiments, the main body portion 600 has an axial length such that, when the handle portion 600 abuts the distal end 202 of the inflow loading assembly 102, the open end 604 extends beyond the coupling members of a medical device extending from the open end 404 of the outflow loading assembly 104.

In embodiments, an inner diameter of the open end 604 is sized to receive a tip of a delivery system. An outer diameter of the main body portion 600 is preferably sized so that the main body portion 600 can pass through the channel collectively defined by the outflow loading assembly 104 and the inflow the inflow loading assembly 102 (and a slot defined by a body the backplate 106).

FIGS. 7A and 7B illustrate an example of a delivery system 700 in accordance with an embodiment hereof. One skilled in the art will realize that FIGS. 7A and 7B illustrate one example of a delivery system and that existing components illustrated in FIGS. 7A and 7B may be removed and/or additional components may be added to the delivery system 700.

As shown in FIG. 7A, delivery system 700 generally comprises a catheter portion 702, a distal portion 704, and a proximal control handle portion 706 by which the distal portion 704 is effectively controlled. The catheter portion 702 is preferably of a length and size so as to permit a controlled delivery of the distal portion 704 to a desired implant location, for example, a patient's heart. In embodiments, the catheter portion 702 includes features to enhance maneuverability, steerability and advancement of the distal portion 704 to the point of implantation. The distal portion 704 preferably provides the means by which an implantable medical device, *e.g.,* a prosthetic valve and stent, can be mounted for delivery to the implant location and further provides for allowing the expansion of the implantable medical device for effective deployment thereof. The control handle portion 706 preferably controls movements as translated to the distal portion 704 by way of elongate structure of the catheter portion 702. Controlled functionality from the control handle portion 706 is preferably provided in order to permit expansion and deployment of the implantable medical device at a desired location, such as a heart valve annulus, and to provide for ease in the delivery and withdrawal of the delivery system through a patient's vasculature.

The catheter portion 702 of the delivery system 700 also preferably comprises an outer shaft 708 that is also operatively connected with the control handle portion 706 and that surrounds one or more inner shafts, e.g., an inner shaft 710 as illustrated in FIG. 7B which is an enlarged view of the distal portion 704, over at least a part of its length. In embodiments, the outer shaft 708 comprises a lubricous inner layer (such as high density polyethylene HDPE or Polytetrafluoroethylene PTFE), braided stainless steel middle layer with a flexible plastic outer layer, such as comprised of Pebax 7233, or Nylon 12. The outer shaft 708 may extend from the control handle portion 706 and may facilitate the advancement and steering of the delivery system along a guide wire and through a patient's vasculature, in particular by improving the pushability of the delivery system 700.

The outer shaft 708 is preferably operatively connected with the control handle portion 706 so as to be movable by operation of the handle control portion and that is connected with a sheath or capsule 712 as further illustrated in FIG. 7B, which is an enlarged view of the distal portion 704. Thus, telescopic movement of the outer shaft 708 by operation of the control handle portion 706 results in the longitudinal translational movement of the capsule 712. The control handle portion 706 is designed, among other things, for controlling the advancement and the withdrawal of the capsule 712.

As illustrated in FIG. 7B, the distal portion 704 may also include one or more attachment members, *e.g.,* a spindle 711, that is coupled to an inner shaft 710. The spindle 711 may be configured to couple the implantable medical device to the catheter portion 702 of the delivery system 700. The spindle 711 can include one or more coupling members, for example, two opposing pockets 715. The pockets 715 can be recesses sized and shaped to closely correspond to the size and shape of coupling members of an implantable medical device, *e.g.,* paddles of a prosthetic heart valve described below in FIGS. 7C and 7D.

A nosecone 714 may be coupled to the inner shaft 710 and/or spindle 711 by a pin 716 at a distal end of the distal portion 704 and operates as the leading feature of delivery system 700. The inner shaft 710 can also include an axial lumen (not shown) extending entirely through at least the inner shaft 710, the spindle 711, pin 716, and the nosecone 714, the purpose of which is for receiving a guidewire in order for the delivery system 700 to be guided along a patient's vasculature to an implant location. The guidewire, not shown, may be used in a conventional manner to guide the delivery system along it and with its distal end guided to its desired implant location.

In embodiments, the implantable medical devices useful with the present disclosure can be a prosthetic valve sold under the trade name CoreValve^{®} available from Medtronic, Inc., Evolut^{™} Pro+ available from Medtronic, Inc., and the like. A non-limiting example of an implantable medical device useful with systems, devices and methods of the present disclosure is illustrated in FIGS. 7B and 7C. In particular, FIG. 7C illustrates a side view of a prosthetic heart valve 750 in a normal or expanded (uncompressed) arrangement. FIG. 7D illustrates the prosthetic heart valve 750 in a compressed arrangement (*e.g.,* when compressively retained within delivery system such as the distal portion 704 of the delivery system 700). The prosthetic heart valve 750 includes a stent or frame 752 and a valve structure 754. The stent 752 can assume any of the forms described above, and is generally constructed so as to be expandable from the compressed arrangement (FIG. 7D) to the uncompressed arrangement (FIG. 7C). In some embodiments, the stent 752 is self-expanding. In other embodiments, the stent 752 is designed to the expanded arrangement by a separate device (*e.g.,* a balloon internally located within the stent 752). The valve structure 754 is assembled to the stent 752 and provides two or more (typically three) leaflets 756. The valve structure 754 can be assembled to the stent 752 in various manners, such as by sewing the valve structure 754 to one or more of the wire segments or commissure posts defined by the stent 752.

The prosthetic heart valve 750 of FIGS. 7C and 7D can be configured to replace or repair an aortic valve. Alternatively, other shapes are also envisioned, adapted to the specific anatomy of the valve to be repaired (*e.g.,* stented prosthetic heart valves in accordance with the present disclosure can be shaped and/or sized for replacing a native mitral, pulmonic, or tricuspid valve). With the example of FIGS. 7C and 7D, the valve structure 754 extends less than the entire length of the stent 752, but in other embodiments can extend along an entirety, or a near entirety, of a length of the stent 754. A wide variety of other constructions are also acceptable and within the scope of the present disclosure. For example, the stent 752 can have a more cylindrical shape in the normal, expanded arrangement.

The stent 752 includes support structures that comprise a number of struts or wire portions 758 arranged relative to each other to provide a desired compressibility and strength to the valve structure 754. The stent 752 can also include one or more paddles 760 that removably couple the prosthetic heart valve 750 to a delivery system, e.g., the delivery system 700. While FIGS. 7C and 7D illustrate paddles 760, one skilled in the art will realize that the paddles 760 can be replaced with other components such as eyelets, loops, slots, or any other suitable coupling member. The paddles 760 (or other portion of the stent 752) can include one or more radiopaque markers that aid in the positioning and orientation of the prosthetic heart valve 750. The struts or wire portions 758 form a lumen having an inflow end 762 and an outflow end 764. The struts or wire portions 758 can be arranged such that the struts or wire portions 758 are capable of transitioning from the compressed arrangement to the uncompressed arrangement. These wires are arranged in such a way that the stent 752 allows for folding or compressing or crimping to the compressed arrangement in which the internal diameter is smaller than the internal diameter when in the uncompressed arrangement. In the compressed arrangement, such the stent 752 with attached valve structure 754 can be mounted onto a delivery system, such as the distal portion 704 the delivery system 700. The stent 752 are configured so that they can be changed to an uncompressed arrangement when desired, such as by the relative movement of one or more sheaths relative to a length of the stent 752.

In embodiments, the struts or wire portions 758 of the stent 752 can be formed of a metal or other material that can be expanded from a compressed arrangement to an uncompressed arrangement by an expansion device, e.g., balloon. In some embodiments, the wires of the support structure of the stent 752 in embodiments of the present disclosure can be formed from a shape memory material such as a nickel titanium alloy (e.g., Nitinol). With this material, the support structure is self-expandable from the compressed arrangement to the normal, expanded arrangement, such as by the application of heat, energy, and the like, or by the removal of external forces (*e.g.,* compressive forces). This stent 752 can also be compressed and re-expanded multiple times without significantly damaging the structure of the stent frame. In addition, the stent 752 of such an embodiment may be laser-cut from a single piece of material or may be assembled from a number of different components or manufactured from a various other methods known in the art.

In embodiments, the stent 752 can generally be tubular support structures having an internal area in which the leaflets 756 can be secured. The leaflets 756 can be formed from a variety of materials, such as autologous tissue, xenograph material, or synthetics as are known in the art. In some embodiments, the leaflets 756 may be provided as a homogenous, biological valve structure, such as porcine, bovine, or equine valves. In some embodiments, the leaflets 756 can be provided independent of one another and subsequently assembled to the support structure of the stent 752. In some embodiments, the stent 752 and the leaflets 756 can be fabricated at the same time, such as may be accomplished using high-strength nano-manufactured NiTi films produced at Advanced Bioprosthetic Surfaces (ABPS), for example. The stent 752 can be configured to accommodate at least two (typically three) of the leaflets 756 but can incorporate more or fewer than three of the leaflets 756.

FIGS. 8 and 9A-9O illustrates an example of a method 800 for loading an implantable medical device using the loading system 100, in accordance with an embodiment hereof. One skilled in the art will realize that FIGS. 8 and 9A-9O illustrate one example of a method using the loading system 100 and that existing operations illustrated in FIGS. 8 and 9A-9O may be removed and/or additional operations may be added to the method 800. In some embodiments, some or all of the operations of loading an implantable medical device are performed in a liquid bath, for example, a cold saline bath. Accordingly, in some embodiments, the materials used for components of the loading system 100 are relatively dimensionally stable when exposed to temperatures at or relatively near the temperature of the liquid bath being used.

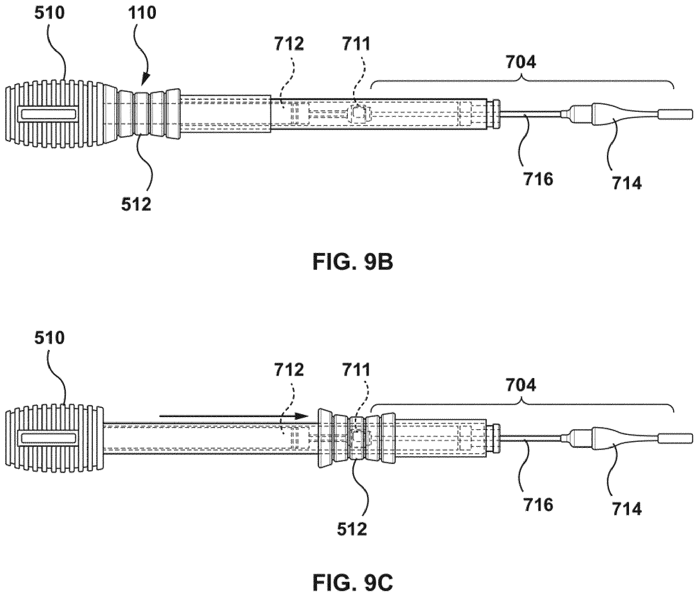

In step 802, a capsule guide can be moved to an unlocked position. For example, as illustrated in FIG. 9A, the locking collar 512 of the capsule guide 110 can be retracted to abut the handle 510, thereby being in an unlocked position. In step 804, the capsule guide 110 can be positioned on a delivery system and advanced. For example, as illustrated in FIG. 9B, the capsule guide 110 in the unlocked position, can be advanced over the distal portion 704 of the delivery system 700 by inserting the distal end of the delivery system into the handle 510 of the capsule guide 100 and advancing the capsule guide 100 proximally until the distal open end 502 of the guide tube 110 is between the spindle 711 of the catheter portion 702 and the nosecone 714.

In step 806, the capsule guide is locked and positioned. For example, as illustrated in FIG. 9C, the locking collar 512 of the capsule guide 110 can be moved toward the distal open end 502. The locking collar 512 locks the capsule guide 110 prior to advancing the capsule guide 110 over the distal portion 704, *e.g.,* a capsule. In embodiments, the locking collar 512 can cause the capsule guide 110 and a flexible capsule to operate as rigid objects, while the locking collar 512 is engaged. The locking collar 512 can compress the two halves of the capsule guide 110 together. The compression of the capsule guide 110 can form a ring that limits a flare of the delivery system from expanding. Additionally, the compression of the locking collar 512 can protect components the delivery system and the capsule guide 110 from damage as the prosthetic heart valve 750 is being loaded.

In step 808, one end of the implantable medical device is inserted into an inflow loading assembly. For example, as illustrated in FIG. 9E, with the backplate 106 inserted, an implantable medical device, *e.g.,* the prosthetic heart valve 750, is inserted into the inflow loading assembly 102. In this example, the inflow end 762 of the prosthetic heart valve 750 can be aligned and inserted into the proximal opening 210 at the proximal end 204 of the inflow loading assembly 102. As discussed above, an inner surface of second portion 208 of the inflow loading assembly 102 can be sized to create an interference fit with the inflow end 762 of the prosthetic heart valve 750. The prosthetic heart valve 750 can be oriented such that the paddles 760 are substantially in a vertical plane and one of the paddles 760 is aligned with the backplate 106 extending from the inflow loading assembly 102. Once inserted, the inflow end 762 of the prosthetic heart valve 750 can be adjacent to and can abut the backplate 106.

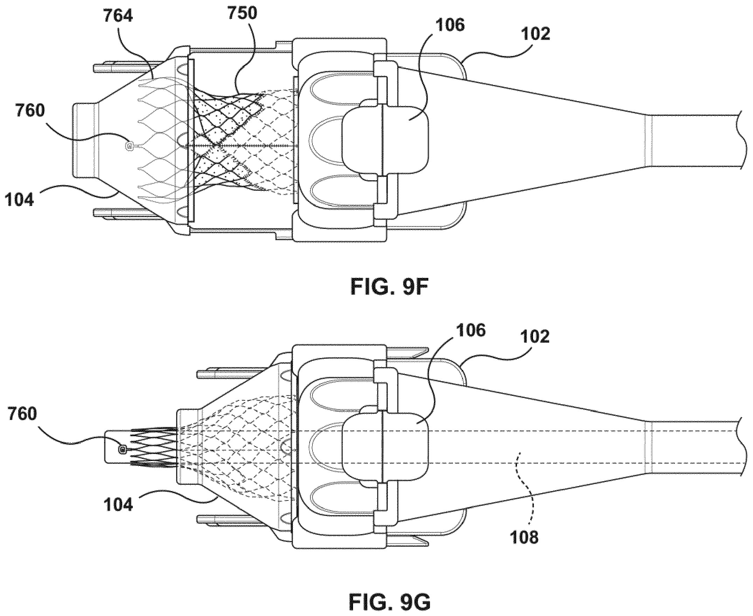

In step 810, an outflow loading assembly is attached to the inflow loading assembly. For example, as illustrated in FIG. 9F, the outflow loading assembly 104 is advanced over the outflow end 764 of the prosthetic heart valve 750 thereby partially compressing the stent 752. The outflow end 764 of the prosthetic heart valve 750 may be advanced along the tapered interior surface of the portion 406 of the outflow loading assembly 104 to compress the outflow end 764 of the prosthetic heart valve 750. The compression occurs by advancing the outflow loading assembly 104, with distal open end 402 facing the prosthetic heart valve 750, towards the prosthetic heart valve 750 seated in the inflow loading assembly 102.

The outflow loading assembly 104 may be advanced over the prosthetic heart valve 750 until the outflow loading assembly 104 couples with the inflow loading assembly 102. The outflow loading assembly 104 can be advanced until the distal open end 402 of the outflow loading assembly 104 is adjacent the proximal end 204 of the inflow loading assembly 102. That is, the engagement tabs 408 of the outflow loading assembly 104 are aligned with the tabs 212 of the inflow loading assembly 102, and the outflow loading assembly 104 is advanced until the engagement tabs 408 and the tabs 212 engage. The backplate 106 can apply an axial force to advance the prosthetic heart valve 750 relative to the outflow loading assembly 104 into a desired final position within the outflow loading assembly 104. For example, the inflow end 762 of the prosthetic heart valve 750 contacts the backplate 106.

In step 812, a tip guide tube is inserted into the inflow loading assembly. For example, as illustrated in FIG. 9G, the tip guide tube 108 can be inserted in the distal opening 209 at the distal end 202 of the inflow loading assembly 102. The tip guide tube 108 can be introduced into the distal opening 209 at the distal end 202 of the inflow loading assembly 102 and advanced within the inflow loading assembly 102 and the outflow loading assembly 104 until the tip guide tube 108 contacts the outflow end 764 of the prosthetic heart valve 750, for example, an inner surface of stent 752 of the prosthetic heart valve 750. Movement of the tip guide tube 108 in a proximal direction through the prosthetic heart valve 750 can properly orient the leaflets 756 of the valve structure 754 such that the risk of damaging the leaflets 756 is reduced while the prosthetic heart valve 750 is further reduced is radial size.

In some embodiments, the tip guide tube 108 can be further advanced to pass through the proximal open end 404 of the outflow loading assembly 104 such that the tip guide tube 108 contacts the portion of the prosthetic heart valve 750 extending beyond through the proximal open end 404 of the outflow loading assembly 104. The tip guide tube 108 contact expands this portion of the outflow end 764 of the prosthetic heart valve 750, spreading open the stent 752. The tip guide tube 108 can contact the portion of prosthetic heart valve 750 extending beyond the proximal open end 404 when handle portion 606 of the tip guide tube 108 is adjacent to or abuts the distal end 202 of the inflow loading assembly 102.

At this point, a user can inspect outflow crowns of the stent 752 to ensure that the outflow crowns are evenly spaced and that the paddles 760 are opposite from each other. If a misalignment exists, a user can manually adjust the stent 752 to achieve the desired configuration. For example, a user can directly inspect the outflow crowns and the paddle(s) 760 directly facing the user, and can indirectly inspect the outflow crowns and the paddle(s) 760 facing away from the user by using a mirror in a loading tray used to load the prosthetic heart valve 750 into the delivery system 700.

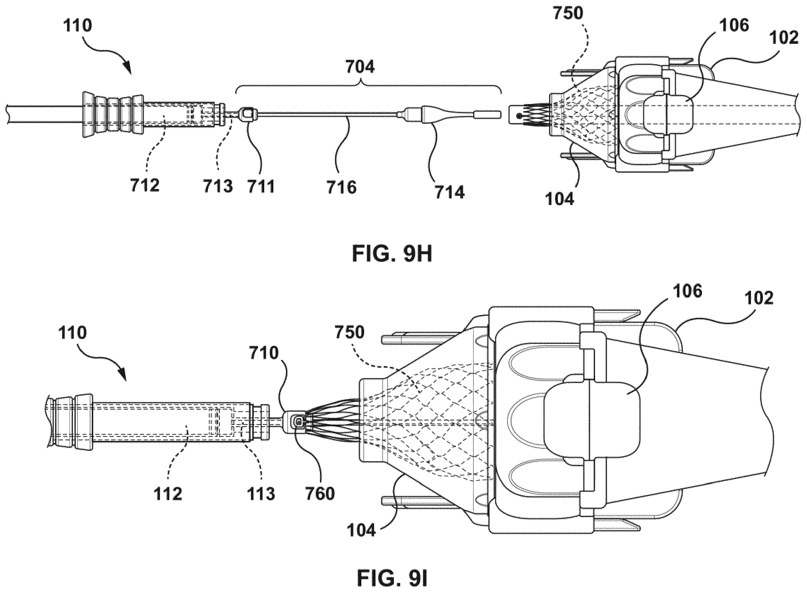

In step 814, the implantable medical device is coupled to the delivery system. For example, as illustrated in FIG. 9H, the prosthetic heart valve 750 and the loading system 100 are positioned over the nosecone 714. That is, the nosecone 714 is inserted into the tip guide tube 108 and advanced. The distal portion 704 is advanced until the paddles 760 of the stent 752 are aligned with the attachment location of the distal portion 704, e.g., the pockets 715 of the spindle 711. The distal portion 704 can be advanced using the capsule guide 110.

Once approximately aligned, the tip guide tube 108 is retracted in order to seat the paddles 760 with the pockets 715 of the spindle 711. That is, the tip guide tube 108 is distally retracted relative to prosthetic heart valve 750, releasing contact between the tip guide tube 108 and the outflow end 764 of the prosthetic heart valve 750 extending beyond the proximal open end 404 of the outflow loading assembly 104. As illustrated in FIG. 9I, the contact release allows outflow portion of the prosthetic heart valve 750 to contract such that the paddles of the stent 752 of engage the pockets 715 of the spindle 711.

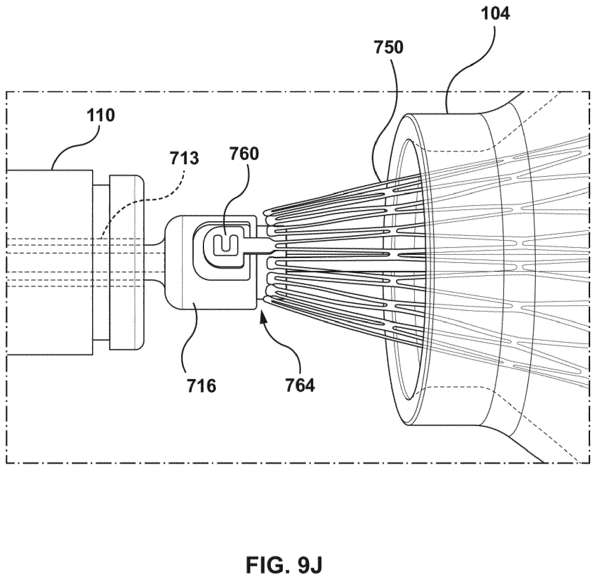

At this point, a user can inspect that the prosthetic heart valve 750 is correctly coupled to the delivery system. For example, a user can inspect that the paddles 760 of the prosthetic heart valve 750 are correctly seated within the pockets 715 of the spindle 711. A user can directly inspect this coupling facing the user and can indirectly inspect the coupling facing away from the user by using the mirror, as illustrated in FIG. 9J. If a misalignment exists, a user can manually adjust the paddles 760 to achieve the desired seating configuration.

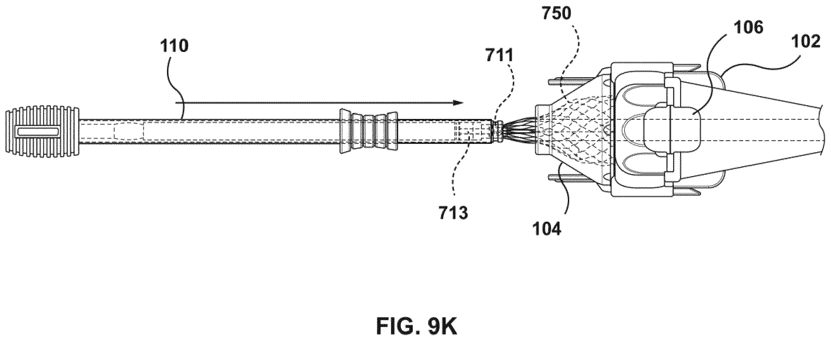

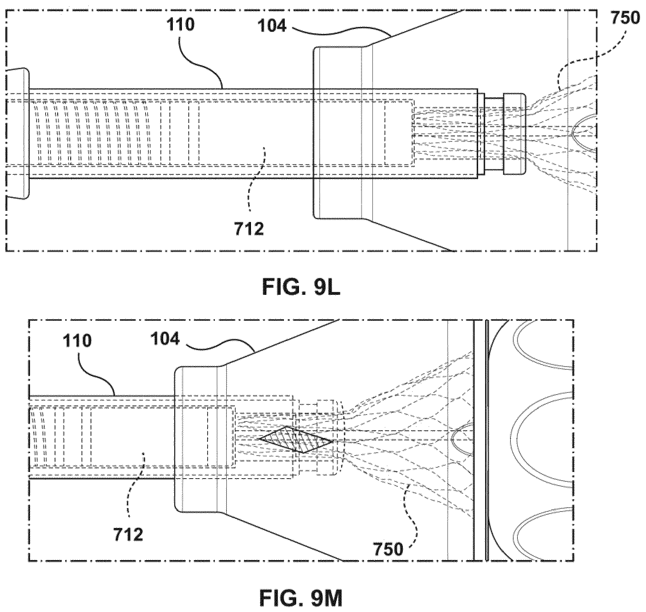

In step 816, the capsule guide is advanced and an end of the implantable medical device is secured within the delivery system. For example, as illustrated in FIGS. 9K, a force is applied the capsule guide 110 is advanced towards the outflow loading assembly 104 until the open distal end 502 covers the spindle 711. Then, as illustrated in FIG. 9L, the control handle portion 706 can be actuated and to advance the capsule 712 until the capsule 712 covers the spindle 711. As such, the capsule 712 secures the paddles 760 within the pockets 715 of the spindle 711. Then, as illustrated in FIG. 9M, the capsule tube 110 can first be advanced over the commissure pads of the prosthetic heart valve 750. Subsequently, the control handle portion 706 can be actuated and to advance the capsule 712 until the capsule 712 covers the commissure pads of the prosthetic heart valve 750.

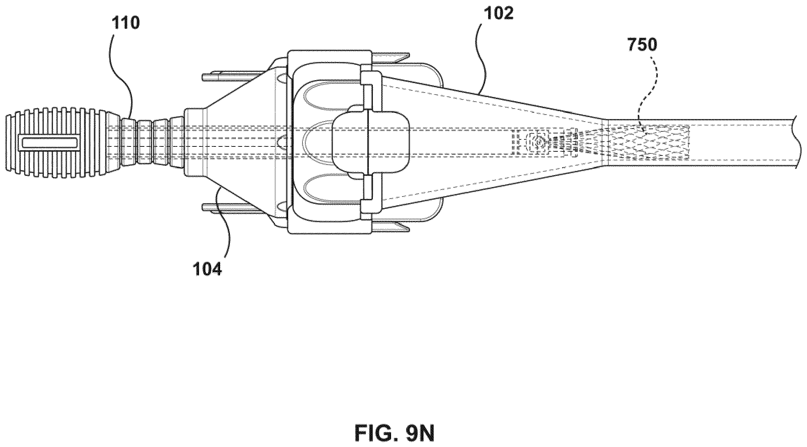

In step 818, the end of the implantable medical device is compressed using the inflow loading assembly. For example, the backplate 106 and the tip guide tube 108 can be removed from the inflow loading assembly 102. Once removed, the capsule guide 110 can be held stationary, and the inflow loading assembly 102 can be advanced over the inflow end of the prosthetic heart valve 750, as illustrated in FIG. 9N. Once the prosthetic heart valve 750 is compressed, the capsule 712 can be advanced to the nosecone 714 thereby covering the prosthetic heart valve 750. The capsule guide 110 can then be removed from the catheter portion 702 by moving the locking collar 512 to the unlock position and sliding the capsule guide 110 over the distal potion 704 and off the distal end of the catheter potion 702.

As discussed above, the inflow loading assembly 102 includes one or more portions that have a non-circular cross-sections and one or more biasing features, as illustrated in FIGS. 2A-2E and FIGS. 3A and 3B that apply a compression force, unevenly, to the exterior surfaces of the implantable medical device. The non-circular cross-sections and/or one or more biasing features are designed to cause overlap of the structural components of the prosthetic heart valve 750 (*e.g.,* struts and crowns) at multiple and select locations. This prevents circumferential pressure build-up by biasing the structural components inwards in predefined areas. As such, the non-circular cross-sections and/or one or more biasing features distribute the overlap of the structural components evenly within interior free spaces of the prosthetic heart valve 750. As such, the non-circular cross-sections and/or one or more biasing features may reduce the occurrence of a concentration of the structural components of the prosthetic heart valve 750 and provide predictability of the location of the overlap. The distributed overlap may allow for safe loading the prosthetic heart valve 750 into lower profile delivery systems.

Although FIGS. 9A-9O described above illustrate the loading system 100 with a prosthetic heart valve, the loading system 100 can be used to load any suitable medical device, for example, implants, stents, and other implantable or temporary prostheses that do not include a valve assembly.

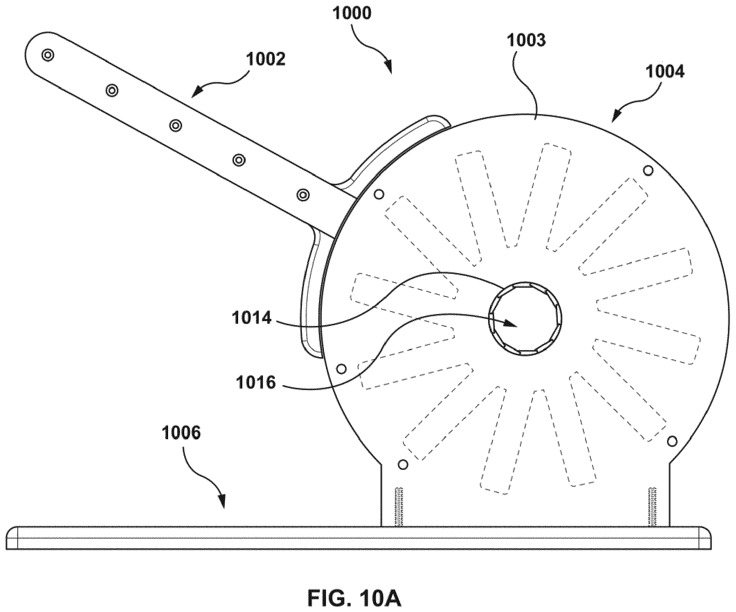

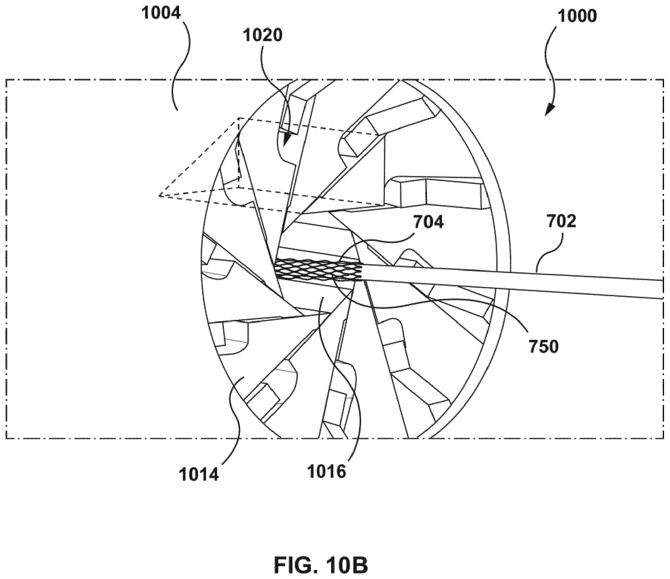

FIGS. 10A and 10B illustrate an example of a crimper 1000 in accordance with an embodiment hereof. One skilled in the art will realize that FIGS. 10A and 10B illustrate one example of a crimper and that existing components illustrated in FIGS. 10A and 10B may be removed and/or additional components may be added to the crimper 1000.

As illustrated in FIG. 10A, the crimper 1000 includes a handle 1002, a crimper housing 1004, and a base 1006. The crimper housing 1004 may include an opening 1008 from a first side 1003 of the crimper housing 1004 to a second side (not shown) of the crimper housing 1004 that is opposite the first side 1003. The opening 1008 can be formed in an approximate circular cross-sectional shape. The opening 1008 can allow access to a crimper chamber 1016 of the crimper 1000 as described in further detail below. The crimper chamber 1016 is formed by a plurality of crimper elements 1014. The handle 1002 extends into the crimper housing 1004 and may couple to one or more actuating mechanisms (not shown), *e.g.,* rods, cams, actuator rings, *etc.*

In embodiments, the one or more actuating mechanisms are coupled to a plurality of crimper elements 1014. The one or more actuating mechanisms may operate to translate the rotational movement of the handle 1002 to the crimper elements 1014. In operation, the crimper elements 1014 are displaced by the movement of the handle 1002. That is, as the handle 1002 is moved, the two cams 1012 may rotate and the rods 1018 may function to translate the rotational motion of the handle 1002 into linear motion of the crimper elements 1014. As such, the crimper elements 1014 of the crimper housing 1004 may function as an iris to decrease or increase the volume of the crimper chamber 1016 through the movement of the handle 1002, as described below in further detail. The crimper chamber 1016 can define a volume that approximates a cylinder. While the crimper chamber 1016 is described above as defining a cylindrically shaped volume, one skilled in the art will realize that the shape and dimension of the lobes can be changed to create a differently shaped volume as required by the implantable medical device being compressed and positioned.

In embodiments, as illustrated in FIG, 10B, the crimper 1000 operates to convert an implantable medical device from its uncompressed arrangement to its compressed arrangement. In operation, the implantable medical device, e.g., prosthetic heart valve 750, is loaded into the crimper chamber 1016 and positioned in a direction that is parallel to the long axis of the base 1006. Portions of delivery system, e.g., catheter 702 of the delivery system 700, can also be positioned and aligned relative to the implantable medical device. Similar to the loading system 100, to address these drawbacks and allow loading in low profile delivery systems, the crimper element 1016 are designed to bias select portions of the implantable medical device towards a central axis of the implantable medical device.

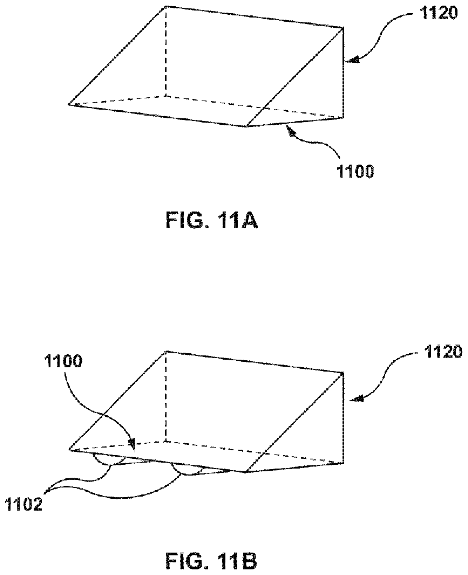

Each of the crimper elements 1014 may include a crimper lobe 1020. FIGS. 11A and 11B illustrated a detailed view of a crimper lobe 1020. The crimper lobe 1020 preferably includes a bottom surface 1100. The bottom surface 1100 defines a portion of the crimper chamber 1016. In embodiments, the bottom surface 1110 of the crimper lobe 1020 includes one or more biasing features 1102 that apply compression force unevenly to the exterior surfaces of the implantable medical device, as illustrated in FIG. 11A and 11B and discussed below in further detail. The biasing features 1102 are designed to cause overlap of the structural components of the implantable medical device at multiple and select locations. As such, the biasing features 1102 distribute the overlap of the structural components evenly within the free space of the implantable medical device, thereby reducing the occurrence of a concentration of the structural components of the implantable medical device. The distributed overlap may allow for safe loading the implantable medical device into lower profile delivery systems. The structural components are crimped asymmetrically. This prevents circumferential pressure build-up by biasing the structural components inwards in predefined areas. This gives predictability in where an overlap of the structural components may occur.

As illustrated in FIG. 11B, the bottom surface 1100 of the crimper lobe 1020 can include two of the biasing features 1102. Each of the biasing features 1102 can be formed as a semi-circular ridge that extends on the bottom surface 1100 from a front of the crimper lobe 1020 to a back of the crimper lobe 1020. In embodiments, when formed as a semi-circular ridge, each of the biasing features 1102 can be formed to any dimensions, e.g., radius and length, in order to provide an asymmetric compression force on the implantable medical device. In some embodiment, a radius of a biasing feature 1102 can range from approximately 0.2 mm to approximately 5 mm. In some embodiments, a length of the biasing feature 1102 can range between approximately 10 mm to approximately 80 mm. In some embodiments, the dimensions of the biasing features 1102 can be constant. In some embodiments, the dimensions of the biasing features 1102 can vary. For example, the radius of biasing features 1102 can increase and/or decrease along the length of the biasing features 1102. That is, each of the biasing features 1102 may have a larger radius at the front of the crimper lobe 1020 as compared to the back of the crimper lobe 1020, or vice versa.

In some embodiments, as illustrated in FIG. 11B, the biasing feature 1102 can extend an entire length of the bottom surface 1100, *e.g.,* from the front of the crimper lobe 1020 to the back of the crimper lobe 1020. In some embodiments, the biasing feature 1102 can extend only a portion of the length of the bottom surface 1100, *e.g.,* from the front of the crimper lobe 1020 to the back of the crimper lobe 1020. While FIG. 11B illustrates the biasing features 1102 as being semi-circular ridge, one skilled in the art will realize that a biasing feature can be formed in any shape and/or size. Likewise, while FIG. 11B illustrates two of the biasing features 1102, one skilled in the art will realize that the crimper lobe 1020 can include any number of biasing features 1102

While the components of the crimper 1000 are described above with relative terms "first," "second," "proximal," and "distal," one skilled in the art will realize that the use of these terms is intended only to identify components of the crimper 1000 and do not define any preferred or ordinal arrangement of the components of crimper 1000.

It should be understood that various embodiments disclosed herein may be combined in different combinations than the combinations specifically presented in the description and accompanying drawings. Each embodiment and each aspect so defined may be combined with any other embodiment or with any other aspect unless clearly indicated to the contrary. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a medical device.

## Claims

1. A system for transitioning an implantable medical device from an uncompressed arrangement to a compressed arrangement, the device comprising:
an inflow loading assembly (102) configured to compress an inflow portion of the implantable medical device as the implantable medical device is advanced through the inflow loading assembly; and
an outflow loading assembly (104) removably coupled to the inflow loading assembly, wherein:
the outflow loading assembly is configured to partially compress an outflow portion of the implantable medical device during coupling to the inflow loading assembly, and
the inflow loading assembly comprises one or more biasing features (150, 226, 326) that are configured to asymmetrically compress the inflow portion of the implantable medical device by biasing structural components of the implantable medical device inwards in predefined areas;
wherein the inflow loading assembly comprises a first portion (206, 306) and a second portion (208, 308) extending therefrom, and wherein at least one of the first portion or the second portion comprises the one or more biasing features; and
wherein at least one of the first portion or the second portion is formed having a non-circular cross-section that provides a non-uniform compression force.

2. The system of claim 1, wherein the biasing features comprise one or more ridges (226) formed on an interior surface of the inflow loading assembly.

3. The system of claim 1 or 2, wherein the biasing features comprise one or more bumps (326) formed on an interior surface of the inflow loading assembly.

4. The system of claim 1, wherein the first portion has a constant cross-sectional area; and/or wherein the first portion and a second portion comprise a common continuous interior surface, in particular wherein the common continuous interior surface having a decreasing diameter from an opening (210, 310) of the second portion to a junction (240) of the second portion and the first portion.

5. The system of claim 1 or 4, wherein the biasing features comprise one or more ridges (226) formed on an interior surface of the inflow loading assembly that extend from an open end of the first portion to an open end of the second portion.

6. The system of any of claims 1, 4 or 5, wherein the biasing features comprise one or more bumps (326) formed on an interior surface of the inflow loading assembly that extend from an open end of the first portion to an open end of the second portion.

7. A system for percutaneously delivering a prosthetic heart valve (750), the prosthetic heart valve being radially self-expandable from a compressed arrangement to an uncompressed arrangement, the system comprising:
a delivery device having a distal portion and a proximal control handle portion by which the distal portion is effectively controlled; and
the loading system according to any preceding claim.

8. A crimper (1000) for altering an implantable medical device from an uncompressed arrangement to a compressed arrangement, the crimper comprising:
a crimper housing (1004) comprising a plurality of crimper elements (1014), the plurality of crimper elements defining a crimper channel, wherein the plurality of crimper elements comprises one or more biasing features (1102) that are configured to asymmetrically compress the implantable medical device; and
a handle (1002) configured to operate the crimper elements, wherein
movement of the handle displaces the plurality of crimper elements, and
the displacement of the plurality of crimper elements decreases a volume of the crimper chamber (1016) to transition the implantable medical device from the uncompressed arrangement to the compressed arrangement,
wherein the biasing features comprise one or more ridges formed on an interior surface of the plurality of crimper elements or one or more bumps formed on the interior surface of the plurality of crimper elements.

## Patentansprüche

1. System zum Umwandeln einer implantierbaren medizinischen Vorrichtung von einer nicht zusammengedrückten Anordnung in eine zusammengedrückte Anordnung, die Vorrichtung umfassend:
eine Zuflussladeanordnung (102), die konfiguriert ist, um einen Zuflussabschnitt der implantierbaren medizinischen Vorrichtung zusammenzudrücken, wenn die implantierbare medizinische Vorrichtung durch die Zuflussladeanordnung hindurch vorgeschoben wird; und
eine Abflussladeanordnung (104), die mit der Zuflussladeanordnung abnehmbar gekoppelt ist, wobei:
die Abflussladeanordnung konfiguriert ist, um einen Abflussabschnitt der implantierbaren medizinischen Vorrichtung während dem Koppeln mit der Zuflussladeanordnung teilweise zusammenzudrücken, und
die Zuflussladeanordnung ein oder mehrere Vorspannmerkmale (150, 226, 326) umfasst, die konfiguriert sind, um den Zuflussabschnitt der implantierbaren medizinischen Vorrichtung durch Vorspannen von Strukturkomponenten der implantierbaren medizinischen Vorrichtung nach innen in vordefinierten Bereichen asymmetrisch zusammenzudrücken;
wobei die Zuflussladeanordnung einen ersten Abschnitt (206, 306) und einen zweiten Abschnitt (208, 308) umfasst, der sich davon erstreckt, und wobei mindestens einer von dem ersten Abschnitt oder dem zweiten Abschnitt die eine oder die mehreren Vorspannfunktionen umfasst; und
wobei mindestens einer von dem ersten und dem zweiten Abschnitt einen nicht kreisförmigen Querschnitt aufweist, der eine ungleichmäßige Druckkraft erzeugt.

2. System nach Anspruch 1, wobei die Vorspannmerkmale eine oder mehrere Rippen (226) umfassen, die auf einer Innenoberfläche der Zuflussladeanordnung ausgebildet sind.

3. System nach Anspruch 1 oder 2, wobei die Vorspannmerkmale eine oder mehrere Erhöhungen (326) umfassen, die auf einer Innenoberfläche der Zuflussladeanordnung ausgebildet sind.

4. System nach Anspruch 1, wobei der erste Abschnitt eine konstante Querschnittsfläche aufweist; und/oder wobei der erste Abschnitt und ein zweiter Abschnitt eine gemeinsame durchgehende Innenoberfläche umfassen, insbesondere wobei die gemeinsame durchgehende Innenoberfläche einen abnehmenden Durchmesser von einer Öffnung (210, 310) des zweiten Abschnitts zu einer Verbindungsstelle (240) des zweiten Abschnitts und des ersten Abschnitts aufweist.

5. System nach Anspruch 1 oder 4, wobei die Vorspannmerkmale eine oder mehrere Rippen (226) umfassen, die auf einer Innenoberfläche der Zuflussladeanordnung ausgebildet sind und sich von einem offenen Ende des ersten Abschnitts zu einem offenen Ende des zweiten Abschnitts erstrecken.

6. System nach einem der Ansprüche 1, 4 oder 5, wobei die Vorspannmerkmale eine oder mehrere Erhöhungen (326) umfassen, die auf einer Innenoberfläche der Zuflussladeanordnung ausgebildet sind und sich von einem offenen Ende des ersten Abschnitts zu einem offenen Ende des zweiten Abschnitts erstrecken.

7. System zum perkutanen Abgeben einer Herzklappenprothese (750), wobei die Herzklappenprothese von einer zusammengedrückten Anordnung zu einer nicht zusammengedrückten Anordnung radial selbst expandierbar ist, das System umfassend:
eine Abgabevorrichtung, die einen distalen Abschnitt und einen proximalen Steuergriffabschnitt aufweist, durch den der distale Abschnitt effektiv gesteuert wird; und
das Ladesystem nach einem der vorstehenden Ansprüche.

8. Crimper (1000) zum Überführen einer implantierbaren medizinischen Vorrichtung von einer nicht zusammengedrückten Anordnung in eine zusammengedrückte Anordnung, der Crimper umfassend:
ein Crimpergehäuse (1004), umfassend eine Vielzahl von Crimperelementen (1014), wobei die Vielzahl von Crimperelementen einen Crimperkanal definiert, wobei die Vielzahl von Crimperelementen ein oder mehrere Vorspannmerkmale (1102) umfasst, die konfiguriert sind, um die implantierbare medizinische Vorrichtung asymmetrisch zusammenzudrücken; und
einen Griff (1002), der konfiguriert ist, um die Crimperelemente zu bedienen, wobei
eine Bewegung des Griffs die Vielzahl von Crimperelementen verschiebt, und
die Verschiebung der Vielzahl von Crimperelementen ein Volumen der Crimperkammer (1016) verringert, um die implantierbare medizinische Vorrichtung von der nicht zusammendrückten Anordnung in die zusammengedrückte Anordnung zu überführen,
wobei die Vorspannmerkmale eine oder mehrere Rippen, die auf einer Innenoberfläche der Vielzahl von Crimperelementen ausgebildet sind, oder eine oder mehrere Erhöhungen umfassen, die auf der Innenoberfläche der Vielzahl von Crimperelementen ausgebildet sind.

## Revendications

1. Système permettant de faire passer un dispositif médical implantable d'un agencement non comprimé à un agencement comprimé, le dispositif comprenant :
un ensemble chargement d'entrée (102) conçu pour comprimer une partie d'entrée du dispositif médical implantable au fur et à mesure que le dispositif médical implantable avance à travers l'ensemble chargement d'entrée ; et
un ensemble chargement de sortie (104) accouplé de manière amovible à l'ensemble chargement d'entrée, dans lequel :
l'ensemble chargement de sortie est conçu pour comprimer partiellement une partie de sortie du dispositif médical implantable pendant l'accouplement avec l'ensemble chargement d'entrée, et
l'ensemble chargement d'entrée comprend une ou plusieurs caractéristiques de sollicitation (150, 226, 326) qui sont conçues pour comprimer de manière asymétrique la partie d'entrée du dispositif médical implantable en sollicitant les composants structurels du dispositif médical implantable vers l'intérieur dans des zones prédéfinies ;
dans lequel l'ensemble chargement d'entrée comprend une première partie (206, 306) et une seconde partie (208, 308) s'étendant à partir de celui-ci, et dans lequel au moins l'une parmi la première partie ou la seconde partie comprend la ou plusieurs caractéristiques de sollicitation ; et
dans lequel au moins l'une parmi la première partie ou la seconde partie est formée avec une section transversale non circulaire qui fournit une force de compression non uniforme.

2. Système selon la revendication 1, dans lequel les caractéristiques de sollicitation comprennent une ou plusieurs crêtes (226) formées sur une surface intérieure de l'ensemble chargement d'entrée.

3. Système selon la revendication 1 ou 2, dans lequel les caractéristiques de sollicitation comprennent une ou plusieurs bosses (326) formées sur une surface intérieure de l'ensemble chargement d'entrée.

4. Système selon la revendication 1, dans lequel la première partie a une surface transversale constante ; et/ou dans lequel la première partie et une seconde partie comprennent une surface intérieure continue commune, en particulier dans lequel la surface intérieure continue commune a un diamètre décroissant à partir d'une ouverture (210, 310) de la seconde partie jusqu'à une jonction (240) de la seconde partie et de la première partie.

5. Système selon la revendication 1 ou 4, dans lequel les caractéristiques de sollicitation comprennent une ou plusieurs crêtes (226) formées sur une surface intérieure de l'ensemble chargement d'entrée qui s'étendent d'une extrémité ouverte de la première partie à une extrémité ouverte de la seconde partie.

6. Système selon l'une quelconque des revendications 1, 4 ou 5, dans lequel les caractéristiques de sollicitation comprennent une ou plusieurs bosses (326) formées sur une surface intérieure de l'ensemble chargement d'entrée qui s'étendent d'une extrémité ouverte de la première partie à une extrémité ouverte de la seconde partie.

7. Système d'administration par voie percutanée d'une valvule cardiaque prothétique (750), la valvule cardiaque prothétique étant radialement auto-expansible d'un agencement comprimé à un agencement non comprimé, le système comprenant :
un dispositif d'administration ayant une partie distale et une partie de poignée de commande proximale par laquelle la partie distale est efficacement commandée ; et
le système de chargement selon l'une quelconque revendication précédente.

8. Sertisseuse (1000) permettant de modifier un dispositif médical implantable d'un agencement non comprimé à un agencement comprimé, la sertisseuse comprenant :
un boîtier de sertisseuse (1004) comprenant une pluralité d'éléments de sertisseuse (1014), la pluralité d'éléments de sertisseuse définissant un canal de sertisseuse, dans laquelle la pluralité d'éléments de sertisseuse comprend une ou plusieurs caractéristiques de sollicitation (1102) qui sont conçues pour comprimer de manière asymétrique le dispositif médical implantable ; et
une poignée (1002) conçue pour faire fonctionner les éléments de sertisseuse, dans laquelle
le mouvement de la poignée déplace la pluralité d'éléments de sertisseuse, et
le déplacement de la pluralité d'éléments de sertisseuse diminue un volume de la chambre de sertisseuse (1016) pour la transition du dispositif médical implantable de l'agencement non comprimé à l'agencement comprimé,
dans laquelle les caractéristiques de sollicitation comprennent une ou plusieurs crêtes formées sur une surface intérieure de la pluralité d'éléments de sertisseuse ou une ou plusieurs bosses formées sur la surface intérieure de la pluralité d'éléments de sertisseuse.
